# EUROPEAN PATENT SPECIFICATION

(11) **EP 0 881 288 B1**
(45) Date of publication and mention of the grant of the patent: **16.09.2009**
(21) Application number: 98109516.9
(22) Date of filing: 26.05.1998
(51) Int. Cl.: C12N 15/00, A01K 67/027, C07K 14/47, C12N 15/62, C07K 16/18, C12Q 1/68

(54) **Purification of higher order transcription complexes from transgenic non-human animals**
Reinigung von Transkription-Komplexen höherer Ordnung aus nicht-menschlichen transgenen Tieren
Purification de complexes transcriptionnels d'ordre supérieur à partir d'animaux transgéniques non humains

(30) Priority: 26.05.1997 EP 97108433
(43) Date of publication of application: 02.12.1998
(73) Proprietor: Sanofi-Aventis Deutschland GmbH, 65929 Frankfurt am Main (DE)
(72) Inventor: Kirschbaum, Bernd, Dr., 55122 Mainz (DE); Berglund, Erick, Dr., 65185 Wiesbaden (DE); Meisterernst, Michael, Dr., 82223 Eichenau (DE); Polites, Greg, Dr. HMR, Inc., Bridgewater, New Jersey 08807-0800 (US)
(74) Representative: Bösl, Raphael Konrad

(56) References cited:
- EP-A- 0 451 700
- WO-A-93/15213
- WO-A-96/09311
- CHIANG, C.M. ET AL.: "Unique TATA-binding protein-containing complexes and cofactors involved in transcription by RNA polymerases II and III" EMBO JOURNAL., vol. 12, no. 7, 1993, pages 2749-2762, XP002047769 EYNSHAM, OXFORD GB
- ZHOU, Q. ET AL.: "Factors (TAFs) required for activated transcription interact with TATA box-binding protein conserved core domain" GENES & DEVELOPMENT, vol. 7, no. 2, February 1993, pages 180-187, XP002047770
- TRIVEDI, A. ET AL.: "TATA-binding protein is limiting for both TATA-containing and TATA-lacking RNA polymerase III promoters in Drosophila cells" MOLECULAR AND CELLULAR BIOLOGY, vol. 16, no. 12, December 1996, pages 6909-6916, XP002047772 WASHINGTON US
- COLGAN, J. & MANLEY, J.L.: "TFIID can be rate limiting in vivo for TATA-containing, but not TATA-lacking, RNA polymerase II promoters" GENES & DEVELOPMENT, vol. 6, no. 2, February 1992, pages 304-315, XP002047771
- WU, S.Y. & CHIANG, C.M.: "Establishment of stable cell lines expressing potentially toxic proteins by tetracycline-regulated and epitope tagging methods" BIOTECHNIQUES., vol. 21, no. 4, October 1996, pages 718-725, XP002047773 NATICK US
- BRYANT, G.O. ET AL.: "Radical mutations reveals TATA-box binding protein surfaces required for activated transcription in vivo" GENES AND DEVELOPEMENT, vol. 10, no. 19, 1 October 1996, pages 2383-2526, XP002079888
- TELLING G C ET AL: "N-TERMINALLY TAGGED PRION PROTEIN SUPPORTS PRION PROPAGATION IN TRANSGENIC MICE" PROTEIN SCIENCE, vol. 6, no. 4, April 1997, pages 825-833, XP000675217
- LANDEL C P: "THE PRODUCTION OF TRANSGENIC MICE BY EMBRYO MICROINJECTIOM" GENETIC ANALYSIS TECHNIQUES AND APPLICATIONS, vol. 8, no. 3, 1 May 1991, pages 83-94, XP000272370

### SEQUENCE LISTING

(1) GENERAL INFORMATION:
   (i) APPLICANT:
      (A) NAME: Hoechst Aktiengesellschaft
      (B) STREET:-
      (C) CITY: Frankfurt
      (D) STATE: -
      (E) COUNTRY: Germany
      (F) POSTAL CODE (ZIP): 65926
      (G) TELEPHONE: 069-305-7072
      (H) TELEFAX: 069-35-7175
      (I) TELEX:-
   (ii) TITLE OF INVENTION: Purification of higher order transcription complexes from transgenic non-human animals
   (iii) NUMBER OF SEQUENCES: 17
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS:
      (A) LENGTH: 12 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Peptide
      (B) LOCATION: 1..12
   (xi) SEQUENCE DESCRIPTION: SEQ ID NO: 1:
   Met Gly Tyr Pro Tyr Asp Val Pro Asp Tyr Ala Val
   1 5 10
(2) INFORMATION FOR SEQ ID NO: 2:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Peptide
      (B) LOCATION: 1..11
   (xi) SEQUENCE DESCRIPTION: SEQ ID NO: 2:
   Met Gly Tyr Pro Tyr Asp Val Pro Asp Tyr Ala
   1 5 10
(2) INFORMATION FOR SEQ ID NO: 3:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Peptide
      (B) LOCATION: 1..10
   (xi) SEQUENCE DESCRIPTION: SEQ ID NO: 3:
   Gly Tyr Pro Tyr Asp Val Pro Asp Tyr Ala
   1 5 10
(2) INFORMATION FOR SEQ ID NO: 4:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Peptide
      (B) LOCATION: 1..9
   (xi) SEQUENCE DESCRIPTION: SEQ ID NO: 4:
   Tyr Pro Tyr Asp Val Pro Asp Tyr Ala
   1               5
(2) INFORMATION FOR SEQ ID NO: 5:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: exon
      (B) LOCATION: 1..22
   (xi) SEQUENCE DESCRIPTION: SEQ ID NO: 5:
      GGAGCAACCG CCTGCTGGGT GC 22
(2) INFORMATION FOR SEQ ID NO: 6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: exon
      (B) LOCATION: 1..21
   (xi) SEQUENCE DESCRIPTION: SEQ ID NO: 6:
      CCTGTGTTGC CTGCTGGGAC G 21
(2) INFORMATION FOR SEQ ID NO: 7:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: exon
      (B) LOCATION: 1..21
   (xi) SEQUENCE DESCRIPTION: SEQ ID NO: 7:
      GGAGACTGAA GTTAGGCCAG C 21
(2) INFORMATION FOR SEQ ID NO: 8:
   (i) SEQUENCE CHARACTERISTICS:
      (A) LENGTH: 76 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: exon
      (B) LOCATION: 1..76
   (xi) SEQUENCE DESCRIPTION: SEQ ID NO: 8:
   GCGGCACCAG GCCGCTGCTG TGATGATGAT GATGATGGCT GCTGCCCATG ACTGCGTAAT 60
   GCGGTCATGA CGCTTT 76
(2) INFORMATION FOR SEQ ID NO: 9:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: exon
      (B) LOCATION: 1..75
   (xi) SEQUENCE DESCRIPTION: SEQ ID NO: 9:
   GAAGGGGGTG GGGGAGGCAA GGGTACATGA GAGCCATTAC GTCGTCTTCC TGAATCCCTT 60
   TAGCCGCTTT GCTCG 75
(2) INFORMATION FOR SEQ ID NO: 10:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: exon
      (B) LOCATION: 1..22
   (xi) SEQUENCE DESCRIPTION: SEQ ID NO: 10:
      CCCTATGACG TCCCGGATTA CG 22
(2) INFORMATION FOR SEQ ID NO: 11:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: exon
      (B) LOCATION: 1..22
   (xi) SEQUENCE DESCRIPTION: SEQ ID NO: 11:
      GTGGAGTGGT GCCCGGCAAG GG 22
(2) INFORMATION FOR SEQ ID NO: 12:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Peptide
      (B) LOCATION: 1..19
   (xi) SEQUENCE DESCRIPTION: SEQ ID NO: 12:
   Met Gly Ser Ser His His His His His His Ser Ser Gly Leu Val Pro
   1               5                   10                  15
   Arg Gly Cys
(2) INFORMATION FOR SEQ ID NO: 13:
   (i) SEQUENCE CHARACTERISTICS:
      (A) LENGTH: 1310 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: exon
      (B) LOCATION: 1..1310
   (xi) SEQUENCE DESCRIPTION: SEQ ID NO: 13:
   CCATGGGCTA TCCCTATGAC GTCCCGGATT ACGCAGTCAT GGGCAGCAGC CATCATCATC     60
   ATCATCACAG CAGCGGCCTG GTGCCGCGCG GCAGCCATAT GGATCAGAAC AACAGCCTGC    120
   CACCTTACGC TCAGGGCTTG GCCTCCCCTC AGGGTGCCAT GACTCCCGGA ATCCCTATCT    180
   TTAGTCCAAT GATGCCTTAT GGCACTGGAC TGACCCCACA GCCTATTCAG AACACCAATA    240
   GTCTGTCTAT TTTGGAAGAG CAACAAAGGC AGCAGCAGCA ACAACAACAG CAGCAGCAGC    300
   AGCAGCAGCA GCAGCAACAG CAACAGCAGC AGCAGCAGCA GCAGCAGCAG CAGCAGCAGC    360
   AGCAGCAGCA GCAGCAGCAA CAGGCAGTGG CAGCTGCAGC CGTTCAGCAG TCAACGTCCC    420
   AGCAGGCAAC ACAGGGAACC TCAGGCCAGG CACCACAGCT CTTCCACTCA CAGACTCTCA    480
   CAACTGCACC CTTGCCGGGC ACCACTCCAC TGTATCCCTC CCCCATGACT CCCATGACCC    540
   CCATCACTCC TGCCACGCCA GCTTCGGAGA GTTCTGGGAT TGTACCGCAG CTGCAAAATA    600
   TTGTATCCAC AGTGAATCTT GGTTGTAAAC TTGACCTAAA GACCATTGCA CTTCGTGCCC    660
   GAAACGCCGA ATATAATCCC AAGCGGTTTG CTGCGGTAAT CATGAGGATA AGAGAGCCAC    720
   GAACCACGGC ACTGATTTTC AGTTCTGGGA AAATGGTGTG CACAGGAGCC AAGAGTGAAG    780
   AACAGTCCAG ACTGGCAGCA AGAAAATATG CTAGAGTTGT ACAGAAGTTG GGTTTTCCAG    840
   CTAAGTTCTT GGACTTCAAG ATTCAGAACA TGGTGGGGAG CTGTGATGTG AAGTTTCCTA    900
   TAAGGTTAGA AGGCCTTGTG CTCACCCACC AACAATTTAG TAGTTATGAG CCAGAGTTAT    960
   TTCCTGGTTT AATCTACAGA ATGATCAAAC CCAGAATTGT TCTCCTTATT TTTGTTTCTG   1020
   GAAAAGTTGT ATTAACAGGT GCTAAAGTCA GAGCAGAAAT TTATGAAGCA TTTGAAAACA   1080
   TCTACCCTAT TCTAAAGGGA TTCAGGAAGA CGACGTAATG GCTCTCATGT ACCCTTGCCT   1140
   CCCCCACCCC CTTCTTTTTT TTTTTTTAAA CAAATCAGTT TGTTTTGGTA CCTTTAAATG   1200
   GTGGTGTTGT GAGAAGATGG ATGTTGAGTT GCAGGGTGTG GCACCAGGTG ATGCCCTTCT   1260
   GTAAGTGCCC CTTCCGGCAT CCCGGAATTC CTGCAGCCCA ACGCGGCCGC              1310
(2) INFORMATION FOR SEQ ID NO: 14:
   (i) SEQUENCE CHARACTERISTICS:
      (A) LENGTH: 4286 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: exon
      (B) LOCATION: 1..4286
   (xi) SEQUENCE DESCRIPTION: SEQ ID NO: 14:
   GAATTCCCCT GCAGGTCACT TAGCGTTGGC CACATAGTAG GTTCTCAAAT ACTTGTTAAT 60
   AAATAAGTTT GTTCGAGAAG CTGGGCAATG ATATTCTACA GCTGGAAGAA GAAACATAAT 120
   GATCTAGTAA TTAGCTCAAT TAAAAATAAA CGTTCTTCTT TCCTCAGAGG AGCATTTCCC 180
   AAGGCCTGCC TTGATAGCCA TCCAAAAAGG CCAAGCTCAT CCAATCTTGC CCTAGATTTA 240
   TGCTAAAATG CAGTTACAAT CGATAGGATG ACAGAAAACG ACAGCACTTA TTTAAATATA 300
   ATAGGCACTT ATTTAAATAG GAGAAGCTGT GACTTCATAG CAAGTGTTGG GGTTAGGAAA 360
   CTGGGTGGAT AAACTTGCTG ATGCTGTAGA TCTTAGCCTC TACATGAGAT CATGTGGAAA 420
   ATCTGAAAGC ATTTTAGGTT CCTTATGTTT GCAATCAAAT AACTGTACAC CTTTTAATTT 480
   AAAAAGTACC ATGAGGCACA CACACACACT CGCAGGAACT TTTTGGCGTA ACAAAACTAG 540
   AATTAGATCT AAAAGCTAAC TGTAGGACTG AGTCTATTCT AAACTGAAAG CCTGGACATC 600
   TGGAGTACCA GGGGGAGATG ACGTGTTACG GGCTTCCATA AAAGCAGCTG GCTTTGAATG 660
   GAAGGAGCCA AGAGGCCAGC ACAGGAGCGG ATTCGTCGCT TTCACGGCCA TCGAGCCGAA 720
   CCTCTCGCAA GTCCGTGAGC CGTTAAGGAG GCCCCCAGTC CCGACCCTTC GCCCCAAGCC 780
   CCTCGGGGTC CCCGGGCCTG GTACTCCTTG CCACACGGGA GGGGCGCGGA AGCCGGGGCG 840
   GAGGAGGAGC CAACCCCGGG CTGGGCTGAG ACCCGCAGAG GAAGACGCTC TAGGGATTTG 900
   TCCCGGACTA GCGAGATGGC AAGGCTGAGG ACGGGAGGCT GATTGAGAGG CGAAGGTACA 960
   CCCTAATCTC AATACAACCT TTGGAGCTAA GCCAGCAATG GTAGAGGGAA GATTCTGCAC 1020
   GTCCCTTCCA GGCGGCCTCC CCGTCACCAC CCCCCCCAAC CCGCCCCGAC CGGAGCTGAG 1080
   AGTAATTCAT ACAAAAGGAC TCGCCCCTGC CTTGGGGAAT CCCAGGGACC GTCGTTAAAC 1140
   TCCCACTAAC GTAGAACCCA GAGATCGCTG CGTTCCCGCC CCCTCACCCG CCCGCTCTCG 1200
   TCATCACTGA GGTGGAGAAG AGCATGCGTG AGGCTCCGGT GCCCGTCAGT GGGCAGAGCG 1260
   CACATCGCCC ACAGTCCCCG AGAAGTTGGG GGGAGGGGTC GGCAATTGAA CCGGTGCCTA 1320
   GAGAAGGTGG CGCGGGGTAA ACTGGGAAAG TGATGTCGTG TACTGGCTCC GCCTTTTTCC 1380
   CGAGGGTGGG GGAGAACCGT ATATAAGTGC AGTAGTCGCC GTGAACGTTC TTTTTCGCAA 1440
   CGGGTTTGCC GCCAGAACAC AGGTAAGTGC CGTGTGTGGT TCCCGCGGGC CTGGCCTCTT 1500
   TACGGGTTAT GGCCCTTGCG TGCCTTGAAT TACTTCCACG CCCCTGGCTG CAGTACGTGA 1560
   TTCTTGATCC CGAGCTTCGG GTTGGAAGTG GGTGGGAGAG TTCGAGGCCT TGCGCTTAAG 1620
   GAGCCCCTTC GCCTCGTGCT TGAGTTGAGG CCTGGCCTGG GCGCTGGGGC CGCCGCGTGC 1680
   GAATCTGGTG GCACCTTCGC GCCTGTCTCG CTGCTTTCGA TAAGTCTCTA GCCATTTAAA 1740
   ATTTTTGATG ACCTGCTGCG ACGCTTTTTT TCTGGCAAGA TAGTCTTGTA AATGCGGGCC 1800
   AAGATCTGCA CACTGGTATT TCGGTTTTTG GGGCCGCGGG CGGCGACGGG GCCCGTGCGT 1860
   CCCAGCGCAC ATGTTCGGCG AGGCGGGGCC TGCGAGCGCG GCCACCGAGA ATCGGACGGG 1920
   GGTAGTCTCA AGCTGGCCGG CCTGCTCTGG TGCCTGGCCT CGCGCCGCCG TGTATCGCCC 1980
   CGCCCTGGGC GGCAAGGCTG GCCCGGTCGG CACCAGTTGC GTGAGCGGAA AGATGGCCGC 2040
   TTCCCGGCCC TGCTGCAGGG AGCTCAAAAT GGAGGACGCG GCGCTCGGGA GAGCGGGCGG 2100
   GTGAGTCACC CACACAAAGG AAAAGGGCCT TTCCGTCCTC AGCCGTCGCT TCATGTGACT 2160
   CCACGGAGTA CCGGGCGCCG TCCAGGCACC TCGATTAGTT CTCGAGCTTT TGGAGTACGT 2220
   CGTCTTTAGG TTGGGGGGAG GGGTTTTATG CGATGGAGTT TCCCCACACT GAGTGGGTGG 2280
   AGACTGAAGT TAGGCCAGCT TGGCACTTGA TGTAATTCTC CTTGGAATTT GCCCTTTTTG 2340
   AGTTTGGATC TTGGTTCATT CTCAAGCCTC AGACAGTGGT TCAAAGTTTT TTTCTTCCAT 2400
   TTCAGGTGTC GTGAGGAATT GCCCGGGGGA TCCATGGGCT ATCCCTATGA CGTCCCGGAT 2460
   TACGCAGTCA TGGGCAGCAG CCATCATCAT CATCATCACA GCAGCGGCCT GGTGCCGCGC 2520
   GGCAGCCATA TGGATCAGAA CAACAGCCTG CCACCTTACG CTCAGGGCTT GGCCTCCCCT 2580
   CAGGGTGCCA TGACTCCCGG AATCCCTATC TTTAGTCCAA TGATGCCTTA TGGCACTGGA 2640
   CTGACCCCAC AGCCTATTCA GAACACCAAT AGTCTGTCTA TTTTGGAAGA GCAACAAAGG 2700
   CAGCAGCAGC AACAACAACA GCAGCAGCAG CAGCAGCAGC AGCAGCAACA GCAACAGCAG 2760
   CAGCAGCAGC AGCAGCAGCA GCAGCAGCAG CAGCAGCAGC AGCAGCAGCA ACAGGCAGTG 2820
   GCAGCTGCAG CCGTTCAGCA GTCAACGTCC CAGCAGGCAA CACAGGGAAC CTCAGGCCAG 2880
   GCACCACAGC TCTTCCACTC ACAGACTCTC ACAACTGCAC CCTTGCCGGG CACCACTCCA 2940
   CTGTATCCCT CCCCCATGAC TCCCATGACC CCCATCACTC CTGCCACGCC AGCTTCGGAG 3000
   AGTTCTGGGA TTGTACCGCA GCTGCAAAAT ATTGTATCCA CAGTGAATCT TGGTTGTAAA 3060
   CTTGACCTAA AGACCATTGC ACTTCGTGCC CGAAACGCCG AATATAATCC CAAGCGGTTT 3120
   GCTGCGGTAA TCATGAGGAT AAGAGAGCCA CGAACCACGG CACTGATTTT CAGTTCTGGG 3180
   AAAATGGTGT GCACAGGAGC CAAGAGTGAA GAACAGTCCA GACTGGCAGC AAGAAAATAT 3240
   GCTAGAGTTG TACAGAAGTT GGGTTTTCCA GCTAAGTTCT TGGACTTCAA GATTCAGAAC 3300
   ATGGTGGGGA GCTGTGATGT GAAGTTTCCT ATAAGGTTAG AAGGCCTTGT GCTCACCCAC 3360
   CAACAATTTA GTAGTTATGA GCCAGAGTTA TTTCCTGGTT TAATCTACAG AATGATCAAA 3420
   CCCAGAATTG TTCTCCTTAT TTTTGTTTCT GGAAAAGTTG TATTAACAGG TGCTAAAGTC 3480
   AGAGCAGAAA TTTATGAAGC ATTTGAAAAC ATCTACCCTA TTCTAAAGGG ATTCAGGAAG 3540
   ACGACGTAAT GGCTCTCATG TACCCTTGCC TCCCCCACCC CCTTCTTTTT TTTTTTTTAA 3600
   ACAAATCAGT TTGTTTTGGT ACCTTTAAAT GGTGGTGTTG TGAGAAGATG GATGTTGAGT 3660
   TGCAGGGTGT GGCACCAGGT GATGCCCTTC TGTAAGTGCC CCTTCCGGCA TCCCGGATAT 3720
   CCTGCAGCCC AACACGGCCG CTCGAGCATG CATCTAGAGA ACGTCACGGC CGCGATCCCC 3780
   CTGTGCCTTC TAGTTGCCAG CCATCTGGTT GTTTGCCCCT CCCCCGTGCC TTCCTTGACC 3840
   CTGGAAGGTG CCACTCCCAC TGTCCTTTCC TAATAAAATG AGGAAATTGC ATCGCATTGT 3900
   CTGAGTAGGT GTCATTCTAT TCTGGGGGGT GGGGTGGGGC AGGACAGCAA GGGGGAGGAT 3960
   TGGGAAGACA ATAGCAGGCA TGCTGGGGAT GCGGTGGGCT CTATGGGTAC CCAGGTGCTG 4020
   AAGAATTGAC CCGGTTCCTC CTGGGCCAGA AAGAAGCAGG CACATCCCCT TCTCTGTGAC 4080
   ACACCCTGTC CACGCCCCTG GTTCTTAGTT CCAGCCCCAC TCATAGGACA CTCAACTTGG 4140
   AGCGGTCTCT CCCTCCCTCA TCAGCCCACC AAACCAAACC TAGCCTCCAA GAGTGGGAAG 4200
   AAATTAAAGC AAGAAGGCTA TTAAGTGCAG AGGGAGAGAA AATGCCTCCA ACATGTGAGG 4260
   AAGTAATGAT AGAAATCATA GAATTC 4286
(2) INFORMATION FOR SEQ ID NO: 15:
   (i) SEQUENCE CHARACTERISTICS:
      (A) LENGTH: 3263 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: exon
      (B) LOCATION: 1..3263
   (xi) SEQUENCE DESCRIPTION: SEQ ID NO: 15:
   ATCGATAAGC TGAGATCCGG CTAGAAACTG CTGAGGGCTG GACCGCATCT GGGGACCATC 60
   TGTTCTTGGC CCTGAGCGGG GCAGGAACTG CTTACCGCAG ATATCCTGTT TGCCCCAATT 120
   CAGCTGTTCC ATCTGTTCTT GGCCCTGAGC GGGGCAGGAA CTGCTTACCA CAGATATCCT 180
   GTTTGGCCCA TATTCAGCTG TCTCTCTGTT CCTGACCTTG ATCTGAACTT CTCTATTCTC 240
   AGTTATGTAT TTTTCCCATG CCTTGCAAAA TGGCGTTACT TAAGCTAGCT TGCCAAACCT 300
   ACGGCTGGGG TCTTTCACGT TTATATCTAT GAGGGGAAGG ACCCAGAGTG GGGAAGCTGG 360
   GATCTTGGGA ACACGCTTCT CTACATGGCA TTGTCTGCAC GGTGGAGTCC GGATCTGAGC 420
   TTGGCTTGGT TTTTAAAACC AGCCTGGAGT AGAGCAGATG GGTTAAGGTG AGTGACCCCT 480
   CAGCCCTGGA CATTCTTAGA TGAGCCCCCT CAGGAGTAGA GAATAATGTT GAGATGAGTT 540
   CTGTTGGCTA AAATAATCAA GGCTAGTCTT TATAAAACTG TCTCCTCTTC TCCTAGCTTC 600
   GATCCAGAGA GAGACCTGGG CGGAGCTGGT CGCTGCTCAG GAACTCCAGG AAAGGAGAAG 660
   CTGAGGTTAC CACGCTGCGA ATGGGTTTAC GGAGATAGCT GGCTTTCCGG GGTGAGTTCT 720
   CGTAAACTCC AGAGCAGCGA TAGGCCGTAA TATCGGGGAA AGCACTATAG GGACATGATG 780
   TTCCACACGT CACATGGGTC GTCCTATCCG AGCCAGTCGT GCCAAAGGGG CGGTCCCGCT 840
   GTGCACACTG GCGCTCCAGG GAGCTCTGCA CTCCGCCCGA AAAGTGCGCT CGGCTCTGCC 900
   AGGACGCGGG GCGCGTGACT ATGCGTGGGC TGGAGCAACC GCCTGCTGGG TGCAAACCCT 960
   TTGCGCCCGG ACTCGTCCAA CGACTATAAA GAGGGCAGGC TGTCCTCTAA GCGTCACCAC 1020
   GACTTCAACG TCCTGAGTAC CTTCTCCTCA CTTACTCCGT AGCTCCAGCT TCACCAGATC 1080
   CTCGAGAACG TCTCCCATGG GCTATCCCTA TGACGTCCCG GATTACGCAG TCATGGGCAG 1140
   CAGCCATCAT CATCATCATC ACAGCAGCGG CCTGGTGCCG CGCGGCAGCC ATATGGATCA 1200
   GAACAACAGC CTGCCACCTT ACGCTCAGGG CTTGGCCTCC CCTCAGGGTG CCATGACTCC 1260
   CGGAATCCCT ATCTTTAGTC CAATGATGCC TTATGGCACT GGACTGACCC CACAGCCTAT 1320
   TCAGAACACC AATAGTCTGT CTATTTTGGA AGAGCAACAA AGGCAGCAGC AGCAACAACA 1380
   ACAGCAGCAG CAGCAGCAGC AGCAGCAGCA ACAGCAACAG CAGCAGCAGC AGCAGCAGCA 1440
   GCAGCAGCAG CAGCAGCAGC AGCAGCAGCA GCAACAGGCA GTGGCAGCTG CAGCCGTTCA 1500
   GCAGTCAACG TCCCAGCAGG CAACACAGGG AACCTCAGGC CAGGCACCAC AGCTCTTCCA 1560
   CTCACAGACT CTCACAACTG CACCCTTGCC GGGCACCACT CCACTGTATC CCTCCCCCAT 1620
   GACTCCCATG ACCCCCATCA CTCCTGCCAC GCCAGCTTCG GAGAGTTCTG GGATTGTACC 1680
   GCAGCTGCAA AATATTGTAT CCACAGTGAA TCTTGGTTGT AAACTTGACC TAAAGACCAT 1740
   TGCACTTCGT GCCCGAAACG CCGAATATAA TCCCAAGCGG TTTGCTGCGG TAATCATGAG 1800
   GATAAGAGAG CCACGAACCA CGGCACTGAT TTTCAGTTCT GGGAAAATGG TGTGCACAGG 1860
   AGCCAAGAGT GAAGAACAGT CCAGACTGGC AGCAAGAAAA TATGCTAGAG TTGTACAGAA 1920
   GTTGGGTTTT CCAGCTAAGT TCTTGGACTT CAAGATTCAG AACATGGTGG GGAGCTGTGA 1980
   TGTGAAGTTT CCTATAAGGT TAGAAGGCCT TGTGCTCACC CACCAACAAT TTAGTAGTTA 2040
   TGAGCCAGAG TTATTTCCTG GTTTAATCTA CAGAATGATC AAACCCAGAA TTGTTCTCCT 2100
   TATTTTTGTT TCTGGAAAAG TTGTATTAAC AGGTGCTAAA GTCAGAGCAG AAATTTATGA 2160
   AGCATTTGAA AACATCTACC CTATTCTAAA GGGATTCAGG AAGACGACGT AATGGCTCTC 2220
   ATGTACCCTT GCCTCCCCCA CCCCCTTCTT TTTTTTTTTT TAAACAAATC AGTTTGTTTT 2280
   GGTACCTTTA AATGGTGGTG TTGTGAGAAG ATGGATGTTG AGTTGCAGGG TGTGGCACCA 2340
   GGTGATGCCC TTCTGTAAGT GCCCCTTCCG GCATCCCGGA ATTCCTGCAG CCCAACGCGG 2400
   CCGCTTCGAG GGATCTTTGT GAAGGAACCT TACTTCTGTG GTGTGACATA ATTGGACAAA 2460
   CTACCTACAG AGATTTAAAG CTCTAAGGTA AATATAAAAT TTTTAAGTGT ATAATGTGTT 2520
   AAACTACTGA TTCTAATTGT TTGTGTATTT TAGATTCCAA CCTATGGAAC TGATGAATGG 2580
   GAGCAGTGGT GGAATGCCTT TAATGAGGAA AACCTGTTTT GCTCAGAAGA AATGCCATCT 2640
   AGTGATGATG AGGCTACTGC TGACTCTCAA CATTCTACTC CTCCAAAAAA GAAGAGAAAG 2700
   GTAGAAGACC CCAAGGACTT TCCTTCAGAA TTGCTAAGTT TTTTGAGTCA TGCTGTGTTT 2760
   AGTAATAGAA CTCTTGCTTG CTTTGCTATT TACACCACAA AGGAAAAAGC TGCACTGCTA 2820
   TACAAGAAAA TTATGGAAAA ATATTCTGTA ACCTTTATAA GTAGGCATAA CAGTTATAAT 2880
   CATAACATAC TGTTTTTTCT TACTCCACAC AGGCATAGAG TGTCTGCTAT TAATAACTAT 2940
   GCTCAAAAAT TGTGTACCTT TAGCTTTTTA ATTTGTAAAG GGGTTAATAA GGAATATTTG 3000
   ATGTATAGTG CCTTGACTAG AGATCATAAT CAGCCATACC ACATTTGTAG AGGTTTTACT 3060
   TGCTTTAAAA AACCTCCCAC ACCTCCCCCT GAACCTGAAA CATAAAATGA ATGCAATTGT 3120
   TGTTGTTAAC TTGTTTATTG CAGCTTATAA TGGTTACAAA TAAAGCAATA GCATCACAAA 3180
   TTTCACAAAT AAAGCATTTT TTTCACTGCA TTCTAGTTGT GGTTTGTCCA AACTCATCAA 3240
   TGTATCTTAT CATGTCTGGA TCC 3263
(2) INFORMATION FOR SEQ ID NO: 16:
   (i) SEQUENCE CHARACTERISTICS:
      (A) LENGTH: 371 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..371
   (xi) SEQUENCE DESCRIPTION: SEQ ID NO: 16:
   Met Gly Tyr Pro Tyr Asp Val Pro Asp Tyr Ala Val Met Gly Ser Ser
   1               5                   10                  15
   His His His His His His Ser Ser Gly Leu Val Pro Arg Gly Ser His
               20                  25                  30
   Met Asp Gln Asn Asn Ser Leu Pro Pro Tyr Ala Gln Gly Leu Ala Ser
           35                  40                  45
   Pro Gln Gly Ala Met Thr Pro Gly Ile Pro Ile Phe Ser Pro Met Met
       50                  55                  60
   Pro Tyr Gly Thr Gly Leu Thr Pro Gln Pro Ile Gln Asn Thr Asn Ser
   65                  70                  75                  80
   Leu Ser Ile Leu Glu Glu Gln Gln Arg Gln Gln Gln Gln Gln Gln Gln
                   85                  90                  95
   Gln Gln Gln Gln Gln Gln Gln Gln Gln Gln Gln Gln Gln Gln Gln Gln
               100                 105                 110
   Gln Gln Gln Gln Gln Gln Gln Gln Gln Gln Gln Gln Gln Gln Gln Ala
           115                 120                 125
   Val Ala Ala Ala Ala Val Gln Gln Ser Thr Ser Gln Gln Ala Thr Gln
       130                 135                 140
   Gly Thr Ser Gly Gln Ala Pro Gln Leu Phe His Ser Gln Thr Leu Thr
   145                 150                 155                 160
   Thr Ala Pro Leu Pro Gly Thr Thr Pro Leu Tyr Pro Ser Pro Met Thr
                   165                 170                 175
   Pro Met Thr Pro Ile Thr Pro Ala Thr Pro Ala Ser Glu Ser Ser Gly
               180                 185                 190
   Ile Val Pro Gln Leu Gln Asn Ile Val Ser Thr Val Asn Leu Gly Cys
           195                 200                 205
   Lys Leu Asp Leu Lys Thr Ile Ala Leu Arg Ala Arg Asn Ala Glu Tyr
       210                 215                 220
   Asn Pro Lys Arg Phe Ala Ala Val Ile Met Arg Ile Arg Glu Pro Arg
   225 230 235 240
   Thr Thr Ala Leu Ile Phe Ser Ser Gly Lys Met Val Cys Thr Gly Ala
   245 250 255
   Lys Ser Glu Glu Gln Ser Arg Leu Ala Ala Arg Lys Tyr Ala Arg Val
   260 265 270
   Val Gln Lys Leu Gly Phe Pro Ala Lys Phe Leu Asp Phe Lys Ile Gln
   275 280 285
   Asn Met Val Gly Ser Cys Asp Val Lys Phe Pro Ile Arg Leu Glu Gly
   290 295 300
   Leu Val Leu Thr His Gln Gln Phe Ser Ser Tyr Glu Pro Glu Leu Phe
   305 310 315 320
   Pro Gly Leu Ile Tyr Arg Met Ile Lys Pro Arg Ile Val Leu Leu Ile
   325 330 335
   Phe Val Ser Gly Lys Val Val Leu Thr Gly Ala Lys Val Arg Ala Glu
   340 345 350
   Ile Tyr Glu Ala Phe Glu Asn Ile Tyr Pro Ile Leu Lys Gly Phe Arg
   355 360 365
   Lys Thr Thr
   370
(2) INFORMATION FOR SEQ ID NO: 17:
   (i) SEQUENCE CHARACTERISTICS:
      (A) LENGTHs 18 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..18
   (xi) SEQUENCE DESCRIPTION: SEQ ID NO: 17:

## Description

The invention relates to a transgene that comprises DNA encoding for for epitope-tagged TATA-box binding protein (TBP), the production of non-human transgenic animals which express epitope-tagged TBP and the use thereof for affinity purification of (novel) transcription factors and transcription complexes from a variety of eukaryotic tissues and cell-types.

Central to regulation of the eukaryotic transcription event is the stepwise formation and activity of the pre-initiation complex This is a large, multi-subunit complex which is necessary for the correct positioning and initiation of the RNA polymerase II enzyme at the transcription start site. Many of the general transcription factors (GTFs) of this complex have been characterized from eukaryotic nuclei in recent years including TFIIA, TFIIB, TFIID, TFIIE, TFIIF and TFIIH (Zawel and Reinberg (1995) Ann. Rev. Biochem. 64, 533-561; Serizawa et al. (1994) In: Transcription: Mechanisms and regulation. 45-66, Raven press). In TATA-containing promoters, TFIID has a specific affinity for the TATA-box sequence. It is through this sequence recognition that TFIID is the first element to bind the promoter in basal RNA polymerase II transcription, thus nucleating complex formation (Lewin, B. (1990) Cell 61: 1161-1164). The other GTFs bind in a defined, stepwise manner, resulting in a completed pre-initiation complex. Subsequently, this large complex recruits and correctly positions the RNA Polymerase II (Pol II) at the transcription start site to initiate basal transcription. It has become clear that TFIID, itself a multi-subunit complex, plays a key role in the regulation of "activated transcription", loosely defined as elevated levels of mRNA production in the presence of transcriptional activators. Such activators can be naturally occurring enhancer-binding determinants, such as the E-box binding USF (Sawadogo and Roeder (1985) Cell 43: 165-175; Kirschbaum et al. (1992) Mol. Cell. Biol. 12: 5094-5100), or viral factors such as VP16 (Stringer et al. (1990) Nature 345: 783-786).

TFIID is composed of the TATA-binding protein (TBP) and several TBP-associated factors (TAF_{II}s) (In the application "TAF_{II}s" include any kind of transcription factor, transcription activator, transcription inhibitor). As many as 20 different TAF_{II}s have been characterized to date, and TFIID complexes containing different combinations of TAF_{II}s have been observed (Zawel and Reinberg (1995) Ann. Rev. Biochem. 64: 533-561; Hori, R.and Carey, M. (1994) Curr. Opinion Gen. Dev. 4: 236-244). Moreover, different combinations of TAF_{II}slend distinct properties to the TFIID complex. In Drosophila, for example, the pattern formation proteins Hunchback (HB) and Bicoid (BCD) are absolutely reliant on the presence of TAF_{II}60, TAF_{II}110, and TAF_{II}250 in the TFIID complex (Sauer, F. et al. (1995) Science 270, 1783-1788). These TFIID components act as co-activators to the upstream enhancer-bound HB and BCD proteins. The neurogenic factor NTF-1 has been shown to require a minimum complex of TBP. TAF_{II}150 (to which it binds) and TAF_{II}250 for activated transcription, whereas SP1 requires the additional factor TAF_{II}110 for its activation (Chen, J.-L. et al. (1994) Cell 79: 93-105). Another study has identified TAF_{II}28, whose presence is necessary for transcriptional activation by the estrogen and vitamin D3 nuclear receptors (May, M. et al. (1996) EMBO 15: 3093-3104). It is likely that the TAF_{II}s act as transcriptional adapters, transmitting regulatory information from activator/repressor factors to the core initiation complex by way of protein-protein interactions.

As regulated transcription is currently viewed, the expression of individual genes and/or small groups of closely related loci are controlled by definable sets of transcription complex subunits. Though some of the factors are ubiquitous and present in most transcription events, for example GTFs, increasing numbers of gene- and cell-specific elements of regulated transcription are now being described.

There are several proven methods which have been used to identify transcription factors. The early strategies, which uncovered RNA Pol II and the seven GTFs (TFIIA, TFIIB, TFIID, TFIIE, TFIIF, TFIIH, and TFIIJ), mainly involved column fractionation of nuclear preparations from cell lines (Zawel and Reinberg (1995); Serizawa et al. (1994); Roeder, R.G. (1996) TIBS 21: 327-335). These fractions yielded semi-purified proteins with various amounts of transcriptional ability. Most of these fractions appeared to be absolutely necessary for basal transcription. At this point, it was known that the TFIID fraction was responsible for TATA-box recognition. However, attempts to isolate a single protein with TATA-binding ability were not successful. A breakthrough occurred when a single component of yeast was shown to be able to replace TFIID in reconstituted basal transcription assays, which led to the isolation and cloning of a 27kD TATA-binding protein (TBP) (Buratowski, S. et al. (1988) Nature 334: 37-42; Cavallini, B. et al. (1988) Proc. Nat. Acad. Sci. 86: 9803-9809). Use of degenerated primers led to the further identification of genes for the TBP subunit of human (Kao, C.C. et al. (1990) Science 248: 1646-1649; Hoffmann, A. et al. (1990) Nature 346: 387-390; Peterson, M.G. et al. (1990) Science 248: 1625-1630), Drosophila (Hoey, T. et al. (1990) Cell 61: 1179-1186; Muhich, M.L. et al. (1990) Proc. Nat. Acad. Sci: 87, 9148-9152), and mouse TFIID (Tamura, T. et al. (1991) Nuc. Acids Res. 19: 3861-3865).

The availability of the cDNA for TBP from different species made possible a wide range of investigations including the over-expression of these proteins in cell culture. HeLa cell lines were produced which constitutively expressed a TBP protein with an FLAG-tag or the influenca virus hemagglutinin (HA) epitope-tag added to its amino terminus (Zhou, Q. et al. (1993) Genes & Development 7, 180-187; Chiang et al. (1993) EMBO 12, 2749-2762). The FLAG-tag is an epitope consisting of a synthetic sequence of eight amino acids. The HA-tag is a natural epitope with the amino acid sequence SEQ ID NO. 1 "MGYPYDVPDYAV" (one letter code).

Also a shorter peptide from the natural HA tag (10 amino acids from the influenza virus hemagglutinin) has been used for the expression of a fusion-protein containing TBP in a Drosophila cell line (Colgan and Manley (1992), Genes Dev. 6, 304-331; Trivrdi et al., (1996) Mol. Cel. Biol. 16, 6909-6916).

TBP proteins with two epitopes tagged to its amino terminus, the FLAG- and the HA-epitope have been expressed in bacteria (Chiang et al. (1993) EMBO 12: 2749-2762). FLAG-tagged TBP proteins have also been expressed under the control of an inducible promoter (Wu et al., (1996) BioTechniques 21: 718-725). However, monoclonal antibodies against the epitope/epitopes were used to purify TBP-associated complexes from nuclear extracts, thus co-purifying TBP-associated factors of the TFIID complex (TAF_{II}s) (Zhou et al, (1993) Genes Dev. 7: 180-187).

Research continues along these lines in many laboratories, with a recent wave of new TAFs and TAF-interacting factors being identified and characterized from HeLa nuclear extracts and yeast (Hori and Carey (1994) Curr. Op. Gen. Dev. 4: 236-244; Zawel and Reinberg (1995) Ann. Rev. Biochem. 64: 533-561; Roeder, R.G. (1996) Trends Biochem. Sci. 21, 327-335).

From human the TAFs TAF_{II}68, TAF_{II} 55, TAF_{II}30, TAF_{II}28, TAF_{II}20 and TAF_{II}18 are known (Mengus et al. (1995) EMBO 14: 1520-1531; Bertolotti et al. (1996) EMBO 15: 5022-5031; Wu and Chiang (1996) Biotechniques 21: 718-725).

It cannot be overemphasized that although a wealth of factors have been found with these methods, the research is confined to transcription complexes, TAFs and TAF-interacting factors that are particular to the cell-line type or yeast strain that is being used.

The invention relates to a more "universal system", wherein epitope-tagged TBP is applied for the affinity purification of novel transcription complexes and transcription factors, TAFs and TAF-interacting factors in the context of a whole animal, and therefore, from a variety of different eukaryotic tissues and cell types.

The invention relates to a transgenic non-human animal having the ability to overexpress epitope-tagged TATA-box binding protein (TBP). In a further aspect, the use of the transgenic non-human animal, preferably for the identification and isolation of higher order transcription complexes and for identification and isolation of proteins associated in the higher order transcription complex (TAFs and TAF-interacting factors, e.g. transcriptionfactors) is disclosed. In another aspect the invention relates to the preparation of the non-human transgenic animal by introducing a transgene into the germline and/or into somatic cells of the non-human transgenic animal, preferably at a particular stage of development. The description discloses a transgene that can be used for making the transgenic non-human animals.

Thus an embodiment discloses a transgene that encodes an epitope-tagged TBP. Preferably the transgene comprises a first DNA sequence that encodes for one or more epitope-tags and that comprises a second DNA sequence that encodes a TBP. The transgene may comprise further DNA-sequence(s) which encode epitope-tag(s). Preferably, the DNA that encodes TBP is a cDNA. The DNA that encodes TBP could be any naturally occuring DNA, a derivative or a part thereof. The DNA, preferably cDNA can e.g. befrom eukaryotes, including birds, amphibians, reptiles, yeast, C. elegans, mammalians, etc. For example the TBP-encoding DNA from rodents, sheep, dog, cow, pig and primates, human or a part thereof can be used. Preferred is the use of human TBP (hTBP) -cDNA. The invention also comprises the use of any non-naturally occurring DNA, e.g. a TBP-cDNA derivative. A derivative of the DNA might for example have a altered sequence, e.g. a mutated or modified sequence and/or might comprise modified nucleotides. A derivative of the DNA can also be a salt, preferably a physiological tolerable salt.

The transgene comprises one or more DNA sequences that encode for one, two, three, four, five or more epitope-tags. Preferably, the transgene comprise the DNA that encodes for two epitope-tags. The DNA encoding the individual epitope-tags can be located at the 5'- and/or the 3'-end of the DNA that encodes TBP and/or at any suitable position in between the sequence of the DNA that encodes TBP. The DNA encoding individual epitope-tags can be separated and/or arranged in tandem or can be directly adjacent respectively.

As epitope-tag, any natural or synthetic peptide can be used. Each epitope-tag is expressed as fusion protein with TBP, the epitope tag may e.g. be connected directly to the TBP or by a spacer peptide. Preferably, an epitope-tag should offer the opportunity to affinity purify TBP or the fusion protein and to affinity purify proteins which are associated with the TBP or the fusion protein, like TAFs and TAF-interacting factors. Furthermore, an epitope-tag should not destroy the functional activity of the TBP when expressed as fusion protein with TBP. For this purpose preferably short peptides are employed as epitope-tags. They can comprise about 1 to 50 or more amino acids, in particular peptides are employed that comprise 5 to 15 amino acids. Non-limiting examples of peptides that can be used as epitope-tags are the FLAG-epitope, the HA-epitope, multiple Histidine residues (6 to 10 histidine residues or more, preferably 6 histidine residues) (His tag), the Myc tag (Stone et al. (1996) Nature 384: 129-134), streptavidin tags and others. Also shorter peptides of natural epitopes can be used for this purpose. For example the use of the HA-epitope include the use of the epitopes "MGYPYDVPDYA" (SEQ ID NO. 2), "GYPYDVPDYA" (SEQ ID NO. 3), "YPYDVPDYA" (SEQ ID NO. 4) or other peptides derived from the HA-epitope.

In one embodiment the transgene contains the cDNA of human TBP and two DNA-sequences which encode an epitope-tag. Preferably the first DNA sequence encodes the HA epitope, which may serve as an epitope for immunoreaction e.g. with a commercially available monoclonal antibody (Kolodziej and Young (1991) Meth. Enzym. 194: 508-519). Just 3' to the sequence that encodes the HA tag a DNA sequence is located, which encodes for a stretch of 6 histidine residues (His tag), The His tag can form a non-covalent, reversible complex with Ni²⁺ ions. For example, commercially available Ni²⁺ agarose affinity column material is routinely used to purify His-tagged proteins (Hochuli, E. et al. (1987) J. Chromatography 411: 177-184; Janknecht, R. et al. (1991) Proc. Nat. Acad. Sci. 74: 4835). In a special embodiment the transgene comprises the DNA sequence SEQ ID NO. 13. The SEQ ID NO. 13 provides a transgene which encodes for a fusion protein consisting of double tagged hTBP.

In a preferred embodiment the description discloses a transgene that encodes for epitope-tagged TBP and that comprises a promoter for the expression of the fusion protein. The transgene can comprise one or more gene regulatory sequences in addition to a DNA which encodes TBP (e.g. cDNA of TBP or a derivative thereof) and DNA-sequence(s) encoding epitope-tags. Such gene regulatory sequences are, for example, natural or synthetic promoters or parts thereof and/or cis-acting elements (e.g. enhancer, silencer). For this purpose mammalian promoters, for example the promoter of the mouse transferrin gene, the promoter of the neuron-specific enolase (NSE) gene (Forss-Petter, S. et al. (1990) Neuron 5: 187-197) or the promoter of the thymidine kinase gene can be used. Also viral promoters, like for example the promoters of the cytomegalovirus genes or the SV 40 early gene can be used. Preferably inducible or constitutive promoters or derivatives thereof are used. As constitutive promoter for example the promoter of the human elongation factor-1 alpha gene (EF) (Uetsuki, T. et al. (1989) J. Biol. Chem. 264: 5791-5798) can be used. As inducible promoter for example the metallothionine promoter (MT), which has several cis-elements that are responsive to heavy metals (Palmiter, R.D. (1987) Experimentia Supplementum 52: 63-80, Birkhäuser Verlag) can be used. In addition, a promoter of a gene which is expressed e.g. cell-cycle specific, cell-type specific or developmental specific can be used for this purpose.

In a special embodiment the transgene comprises the cDNA of hTBP and DNA-sequences that encode for HA epitope (e.g. 9 amino acids of the natural HA epitope) and His epitope (e.g. 6xhis tag) and a constitutive promoter. For example, TBP expression is controlled by the promoter for human elongation factor-1 alpha (EF) (Uetsuki et al. (1989) J. Biol. Chem. 264: 5791-5798). The EF-promoter is a TATA-less promoter that has been used to express transgenes in mice at moderate but constant levels (Hanaoka, K et al. (1991) Differentiation, 183-189). In a special embodiment of the invention the transgene comprises the DNA sequence which encodes for double-tagged (HA and His epitope) hTBP and the sequence of the EF-promoter, in particular the transgene has the DNA sequence SEQ ID NO. 14.

In another special embodiment the transgene comprises a DNA that encodes for TBP, preferably the cDNA of hTBP and DNA-sequences that encode for HA (e.g. 9 amino acids of the natural HA epitope) and His epitope (e.g. 6xhis) and an inducible promoter. Preferably, the inducible promoter is the metallothionine promoter (MT). For example, in the case that the introduction of an additional TBP encoding sequence into an non-human animals genome and the subsequent expression of TBP or a TBP fusion protein respectively is toxic, this embodiment of the invention will allow the non-human animal to come to term with the transgene encoding for the TBP fusion protein lying silent until the promoter is introduced. The promoter can for example be induced when the non-human animal is full grown or at any developmental stage of interest, e.g. in the case of the MT promoter with an interperitoneal injection containing divalent cations like Zn²⁺, Mg²⁺, Me²⁺ or Cd²⁺. As has been shown in other models, the MT directed gene will then express at elevated levels (Palmiter, R.D. et al. (1982) Cell 29: 701-710). In a particular embodiment the transgene comprises a DNA sequence which encodes for double-tagged hTBP (HA and HIS epitope) and the MT-promoter, in particular the transgene has the DNA sequence SEQ ID NO. 15.

The description discloses a method of making a transgene by connecting the DNA sequence(s) that encode for one or more epitope-tags to the DNA sequence that encodes for the TBP protein.

The description further discloses the use of the transgene. For example the transgene can be used for the preparation of a recombinant vector.
The description also discloses a method of preparing a recombinant vector. This method comprises the integration of the transgene into an appropriate vector, e.g. a vector that contains regulatory sequences. Examples for vectors are expression vectors and retroviruses or derivatives thereof.

The description further discloses the use of recombinant vectors which comprise the transgene. In particular the description discloses the use if of a recombinant vector that comprises a transgene (that e.g. contains a cDNA of TBP or a derivative thereof, in particular hTBP and DNA-sequences encoding epitope-tags, for example HA and His epitope encoding DNA-sequences). The description discloses the use of the vector for introducing the transgene into an eukaryotic cell, in particular for the introduction into a mammalian cell. The description further discloses the use of a vector comprising such transgene for the amplification of the transgenic DNA in bacteria or in eukaryotic cells. An eukaryotic cell into which a vector that contains the transgene has been introduced can also be used for the heterologous/transgenic expression of the transgene. The eukaryotic cell (host cell) might be part of a non-human transgenic animal.

In a major embodiment of the invention the transgene is used for the preparation of a transgenic non-human animal. Therefore, a transgene or a recombinant vector comprising the transgene is introduced into a host cell and/or an non-human animal. In another aspect the invention relates to a transgenic non-human animal that has been produced by introducing the transgene into the non-human animal or a cell thereof. A non-human transgenic animal according to the invention has the ability to overexpress TPB or a fusion protein comprising or consisting of epitope-tagged TBP.

For the preparation of a transgenic animal a non-human animal is used as host animal. Such non-human animal include vertebrates such as rodents, non-human primates, sheep, goat, dog, cow, pig, birds, amphibians, reptiles, etc. Preferred animals are selected from non-human mammalian species of animals, preferably, animals from the rodent family including rats and mice, most preferably mice.

A transgenic non-human animal according to the invention comprises any animal into the genome of which one or more copies of a transgene(s) that directs the expression of or which encodes for TBP or derivatives thereof like a fusion protein consisting of or comprising TBP and epitope-tags. The non-human transgenic animal should have the ability to express the epitope-tagged TBP protein. In a particular embodiment of the invention the non-human transgenic animal can have a transgenic interruption or alteration of the endogenous TBP gene(s) (knock-out animal).

The non-human transgenic animal according to the invention is an animal into which by nonnatural means (i.e. by human manipulation), one or more TBP genes (transgenes according to the invention) that do not occur naturally in the animal e.g., foreign TBP gene or a derivative thereof, like genetically engineered endogenous or foreign TBP gene have been introduced. The non-naturally occurring.TBP gene is called transgene. The transgene may be from the same or a different species as the animal but in any case the transgene is not naturally found in the non-human animal in the configuration and/or at the chromosomal locus conferred by the transgene.

The transgene (transgenetic DNA) may comprise a foreign gene encoding for TBP, i.e. sequences not normally found in the genome of the host animal, like a TBP gene or a cDNA obtained from a different animal species. Alternatively or additionally, a transgene may comprise an endogenous gene encoding for TBP, e.g. DNA sequences that are abnormal in that they have been rearranged or mutated in vitro in order to alter the normal in vivo pattern of expression of the TBP gene, or to alter or eliminate the biological activity of the endogenous TBP. The invention also relates to expression vectors that comprise the transgene and that can be used to prepare the non-human transgenic animal.

The invention further relates to a method of preparing a non-human transgenic animal according to the invention. A non-human transgenic animal according to the invention can be produced by introducing a transgene and/or a vector, e.g. an expression vector which comprises the transgene into the germline or a germline cell respectively and/or into a somatic cell of the non-human animal. For example non-human embryonic target cells at various developmental stages can be used to introduce the transgene of the invention. Different methods can be applied depending on the stage of development of the non-human embryonic target cell(s). Some examples are:
1. Microinjection of non-human zygotes is a preferred method for incorporating a transgene into an animals genome in the course of practicing the invention. Microinjection involves the isolation of non-human embryos at the single cell stage. Therefore a non-human zygote, a non-human fertilized ovum that has not undergone pronuclei fusion or subsequent cell division, is the preferred target cell for microinjection of transgenic DNA (DNA of the transgene). The murine male pronucleus reaches a size of approximately 20 micrometers in diameter, a feature which allows for the reproducible injection of 1-2 picoliters of a solution containing transgenic DNA. The use of a non-human zygote for introduction of a transgene has the advantage that, in most cases, the injected transgenic DNA will be incorporated into the host non-human animal's genome before the first cell division (Brinster, et al. (1985) Proc. Natl. Acad. Sci. 82: 4438-4442). As a consequence, all cells of the resultant non-human transgenic animals (founder animals) stably carry an incorporated transgene at a particular genetic locus, referred to as a transgenic allele. The transgenic allele demonstrates Mendelian inheritance: half of the offspring resulting from the cross of a non-human transgenic animal with a non-transgenic animal will inherit the transgenic allele, in accordance with Mendel's rules of random assortment.
   Commonly used procedures for manipulation of embryo and for microinjection of transgenic DNA are described in detail in Transgenic Animal Technology - A Laboratory Handbook" edited by Carl A. Pinkert, Academic Press, Inc. (1994).
2. Viral integration can also be used to introduce a transgene according to the invention into an animal. The developing non-human embryos are cultured in vitro to the developmental stage known as a blastocyst. At this time, the blastomeres may be infected with vectors containing the transgene (transgenic DNA/DNA-constructs), for example an appropriate viral or retroviral vector can be used for this purpose (Jaenich, R. (1976) Proc. Natl. Sci. (USA) 73: 1260-1264). Transformation or infection of the blastomeres can be enhanced by enzymatic removal of the zona pellucida (Hogan, et al. (1986) Manipulating the Mouse Embryo, Cold Spring Harbor Press, Cold Spring Harbor, N.Y.). If the transgene is introduced into blastomeres via viral vectors, such vectors are typically replication-defective but they remain competent for the integration of transgenic DNA sequences which are linked to vector sequences, into the host animal's genome (Jahner et al. (1985) Proc. Natl. Acad. Sci. (USA) 82: 6927-6931; Van der Putten et al. (1985) Proc. Natl. Acad. Sci. (USA) 82: 6148-6152). Transfection is easily and efficiently obtained by culture of blastomeres on a mono-layer of cells producing the transgene-containing vector (Van der Putten et al. (1985) Proc. Natl. Acad. Sci. (USA) 82: 6148-6152; Stewart et al. (1987) EMBO 6: 383-388).
   Alternatively, infection may be performed at a later stage, such as a blastocoele (Jahner, D. et al. (1982) Nature 298: 623-628). In any event, most non-human transgenic founder animals produced by retoviral or viral integration into only a subset of all the cells that form the non-human transgenic founder animal. Moreover, multiple (retro)viral integration events may occur in a single founder animal, generating multiple transgenic alleles which will segregate in future generations of offspring. Introduction of a transgene into germline cells by this method is possible but probably occurs at a low frequency (Jahner, D. et al. (1982) Nature 298:623-628).
   However, once a transgene has been introduced into non-human germline cells by this method, non-human offspring may be produced in which the transgenic allele is present in all of the animal's cells, i.e., in both somatic and germline cells.
3. non-human Embryonic stem (ES) cells can also serve as target cells for introduction of a transgene according to the invention into animals. non-human ES cells are obtained from pre-implantation non-human embryos that can be cultured in vitro (Evens, M.J. et al. (1981) Nature 292: 154-156; Bradley, M.O. et al. (1984) Nature 309: 255-258; Gossler, et al. (1986) Proc. Natl. Acad. Sci. (USA) 83: 9065-9069; Robertson et al. (1986) Nature 322: 455-448; Robertson, E.J., in Teratocarcinomas and Embryonic Stem Cells: A Practical Approach, Robertson, E.J., ed., IRL Press, Oxford (1987), pages 71-112). non-human ES cells, which are commercially available (from, e.g., Genome Systems, Inc., St. Louis, MO), can be transformed with one or more transgenes by established methods (Lovell-Badge, R.H., in Teratocarcinomas and Embryonic Stem Cells: A Practical Approach, Robertson, E.J., ed., IRL Press, Oxford (1987), pages 153-182). Transformed non-human ES cells can be combined with an animal blastocyst, whereafter the non-human ES cells colonize the embryo and contribute to the germline of the resulting animal, which is a chimera (composed of cells derived from two or more animals) (Jaenisch, R. (1988) Science 240: 1468-1474; Bradley, A., in Teratocarcinomas and Embryonic Stem Cells: A Practical Approach, Robertson, E.J., ed., IRL Press, Oxford (1987), pages 113-151). Again, once a transgene has been introduced into non-human germline cells by this method, offspring may be produced in which the transgenic allele is present in all of the non-human animal's cells, i.e., in both somatic and germline cells.

However it occurs, the initial introduction of a transgene is a non-Mendelian event. However, a transgene of the invention may be stably integrated into non-human germ line cells and transmitted to offspring of the non-human transgenic animal as Mendelian loci. Germline integration is essential for the production of non-human transgenic animals that can transmit the genetic information to their progeny in a Mendelian fashion and in order to utilize these non-human transgenic animals as perpetual animal models.

Other transgenic techniques result in mosaic non-human transgenic animal, in which some cells carry the transgene and other cells do not. In mosaic non-human transgenic animals in which germ line cells do not carry the transgene, transmission of the transgene to offspring does not occur. Nevertheless, mosaic non-human transgenic animals are capable of demonstrating phenotypes associated with the transgene and may be used for affinity purification of particular transcription complexes, TAFs and TAF interacting factors. The invention relates to mosaic non-human transgenic animals that contain the described transgene of the invention.

The description discloses transgenically introduced mutations, this comprises null ("knock-out") alleles in which the DNA sequence encoding for the speciesspecific TBP is deleted and/or substituted by a genetically altered TBP sequence (e.g. a transgene according to the invention) of the same or a different species under the control of the promoter(s)/enhancer(s) of choice.

The invention relates to a non-human transgenic animal into which a transgene comprising the DNA encoding epitope-tagged TBP, if nessecary, in the context of a constitutive or an inducible promoter has been introduced. In particular, the invention relates to a non-human transgenic animal into which a transgene that comprises the DNA encoding HA and His epitope-tagged hTBP and the DNA sequence of the EF promoter has been introduced. In another special embodiment the invention relates to a non-human transgenic animal into which a transgene that comprises the DNA encoding HA and His epitope-tagged hTBP and the DNA sequence of the MT promoter has been introduced. In a special embodiment of the invention the non-human transgenic animal contains the transgene with the sequence SEQ ID NO. 13. Another non-human transgenic animal of the invention contains the transgene with the sequence SEQ ID NO. 14. Another non-human transgenic animal of the invention contains the transgene with the sequence SEQ ID NO. 15. In particular the invention relates to a non-human transgenic animal which has stably integrated into its genome the transgene, for example the DNA sequence SEQ ID NO. 13, SEQ ID No. 14 and/or SEQ ID NO. 15.

Offspring that have inherited the transgene, can be distinguished from littermates that have not inherited the transgene by analysis of genetic material from the offspring for the presence of biomolecules that comprise unique sequences corresponding to sequences of, or encoded by, the transgene. Therefore for example, biological fluids that contain the polypeptides (e.g. the epitope-tagged TBP) uniquely encoded by a transgene according to the invention may be immunoassayed for the presence of the polypeptide encoded by the transgene, e.g. the epitope-tagged TBP. A more simple and reliable means of identifying non-human transgenic offspring comprises obtaining a tissue sample from an extremity of an animal, e.g.. a tail and analyzing the sample for the presence of nucleic acid sequences corresponding to the DNA sequence of a unique portion or portions of the transgene of the invention. The presence of such nucleic acid sequence may be determined by, e.g., hybridization ("Southern". "Northern") analysis with DNA sequences corresponding to unique portions of the transgene, analysis of the products of PCR reactions using DNA sequences in a sample as substrates and oligonucleotides derived from the transgene's DNA sequence, etc.

Therefore, the description also discloses tests wherein possible first generation non-human transgenic animals (Gₒ) as well as all further generation non-human transgenic animals (G₁, G₂, G₃, G₄,....) or non-human animals of transgenic animal lines can be tested for presence of the transgene, e.g. with standard PCR reactions. For this purpose genomic DNA can be extracted from animal tissue, for example from tail tissue after Proteinase K and RNAse treatment. For such PCR reactions the sequence of the primers should correspond to parts of the sequence of the transgene, e.g. within the promoter regions, the DNA region(s) encoding epitope-tag(s) and/or DNA regions unique for the particular TBP construct. With such PCR reactions in mice for example in about 25% of the injected eggs the corresponding PCR product can be detected - i.e. about 25 % of the mice produce positive offspring.

Another method to verify whether or not the non-human animals carry the transgene relates to test for the presence of transgenic mRNA in possible non-human transgenic animals/transgenic animal lines. Initial testing can for example be carried out with S1 analysis, which is very sensitive to small levels of mRNA (Berk, AJ. and Sharp, P.A. (1977) Cell 12, 721)**.** For this purpose, labeled antisense oligonucleotides are hybridized with total RNA that has been isolated from tissue of the non-human animal. Subsequent treatment with S1 nuclease digests all single-stranded nucleic acids, DNA and RNA. Double-stranded DNA or DNA-RNA hybrids are left intact. If any transgene mRNA is present, it hybridizes with the antisense oligonucleotide and thus "protects" it from S1 nuclease digestion.

The test comprises that the presence of transgenic mRNA is detected in tissue preparations, e.g. in preparations of total liver mRNA by S1 protection assays. Antisense oligonucleotides can be synthesized which are complementary to a part of the transgenic mRNA (e.g. the mRNA corresponding to the DNA sequence of epitope-tagged TBP). Oligonucleotides are labeled, e.g. 5'end-labeled, for example radioactively with ³²S_{,} ³³P , ³⁵P, ³H or ¹⁴C or with fluorescence markers or other types of markers, like biotin or digoxygenin. Labeled oligonucleotides are mixed with mRNA from the transgenic mouse under selective hybridizing conditions according to standard protocols (Sambrook et al., 1989). The mixture is then treated with S1 nuclease. A short region of non-matching sequence, e.g. at the 3'end of the oligonucleotide should always be digested, and provides an internal control to show that the S1 nuclease indeed digests all available single-stranded nucleic acid. In the presence of transgenic mRNA, the labeled oligonucleotide should be protected from digestion and its presence and size could be easily determined e.g. by sequencing-style denaturing gel (e.g. by 8M urea PAGE) electrophoresis. The presence of undigested, labeled oligonucleotide can then be detected e.g. by exposing the gel to a film. Absence of transgenic mRNA would give no band, as the unprotected oligonucleotide would be digested by the S1 nuclease. The test includes that different tissues and cells relating to different cell types were prepared from the animals and tested for the presence of transgenic mRNA.

Another embodiment relates to test non-human transgenic animals with Northern analysis. Most preferably non-human transgenic animals that were found to be positive for transgenic mRNA by PCR or S1 nuclease mapping were further tested with Northern analysis (McMaster, G.K. and Carmichael (1977) Proc. Nat. Acad. Sci. 74: 4835). For this purpose total RNA from different tissues and/or cell types, can be isolated and size separated e.g. under denaturing conditions on an agarose gel. The RNA can then be bound for example onto a membrane or filter using e.g. capillary transfer and UV cross-linked. The bound RNA can be probed using conventional Northern blot conditions with a labeled probe corresponding to the coding region of the transgene or parts thereof, e.g. with a DNA-probe corresponding to the 5'end of the transgene according to SEQ ID NO. 13. Such DNA-probes have preferably a length of about 20 to 1000 basepairs (bp). Most preferably they have a length of about 100, 200, 300, 400 and 500 bp. If nessecary, excess of the labeled probe is rinsed away and the membrane is exposed to film. The probes can be labeled as described above for oligonucleotides. Oligonucleotides may sometimes also be used for those Northern blot experiments.

A non-human transgenic animal, preferably such non-human transgenic animal which has been positive in any other molecular biological test, are being tested for presence of the transgenic TBP protein with specific immunoreaction e.g. with Western blotting or ELISA. Therefore from tissue or particular cells of the non-human animals, preferably from liver tissue or any other soft tissue nuclei can be isolated by standard procedures, e.g. on an ultracentrifuged sucrose gradient. From such nuclei, total nuclear protein can be collected, e.g. by treating the nuclei with high salt conditions (e.g. 400mM KCl) and non-ionic surfactant (e.g. NP-40). Afterwards nuclear protein can be size separated e.g. with appropriate denaturing gel electrophoresis, preferably by SDS-PAGE. Such gels can be transferred, e.g. electro-transferred on a solid surface, e.g. on membranes or filters, preferably onto nitrocellulose membrane. Membranes or filters can then be preblocked and probed with suitable antibodies according to standard protocols (Sambrook et al. "Molecular Cloning" Second Edition (1989), Cold Spring Harbor Laboratory Press).

For such purpose polyclonal and/or monoclonal antibodies can be applied, e.g. generated in mouse, rabbit, rat, sheep, goat, horse, birds etc. The detection could be performed directly with antibodies that recognize the TBP fusion protein and which are coupled to an enzyme or a marker, e.g. alkaline phosphatase or flourescence marker or biotin or digoxigenin or radiolabel. The detection can also be performed indirectly by using a second antibody which recognizes a conserved region of the primary antibody, for example when the first antibody is generated in mouse the second antibody has to be an anti-mouse antibody generated for example in sheep. Such second antibody can also be coupled to an enzyme or other markers for detection.

The description further discloses the use of the transgene or a part thereof or the encoded fusion protein or a part thereof for the production of antibodiese which bind the epitope-tagged TBP encoded by the transgene, e.g. to the preparation of monoclonal or polyclonal antibodies.

Antibodies with respect to the invention are antibodies that recognize one or more epitope(s) of the TBP fusion protein. Such antibodies could be directed against individual epitope(s) belonging to the TBP and/or could be directed against the epitope-tag(s). One embodiment discloses an antibody which recognizes the amino-terminal region of the TBP fusion protein. Another embodiment relates to an antibody which recognize the carboxy-terminal region of the TBP fusion protein. Another embodiment relates to an antibody which recognize the epitope neighbouring the part of the amino acid sequence where the TBP and the epitope-tag(s) and/or where the two epitope-tags are connected together.

One embodiment discloses an antibody that recognizes an epitope of the fusion protein that consists of hTBP and two epitope-tags. In particular, the antibody recognizes an epitope of the fusion protein consisting of His- and HA- tag and hTBP. Most preferably the antibody recognizes an epitope(s) at the amioterminal end of HA- and His-tagged TBP. A special embodiment relates to an antibody that recognizes the amino acid sequence SEQ ID NO. 16 or an epitope of the correctly folded fusion protein having the amino acid sequence SEQ ID NO. 16 or a part thereof. The description discloses an antibody (anti-TBP antibodies) that recognizes the amino acid sequence SEQ ID NO. 17 or the correctly folded epitope thereof, preferably in the context of a TBP fusion protein.

Antibodies can be polyclonal or monoclonal. An antibody can be generated in all species of non-human animals, preferably from mouse, rat, rabbit, sheep, goat, horse, birds (e.g. from their eggs). Such antibody can be generated according to standard protocols (Hurlow and Lane "Antibodies: A Laboratory Manual" (1988) Cold Spring Harbor Press). Antibodies can, if nessecary be affinity purified using the original immunization peptide (epitope). A special embodiment of the invention relates to polyclonal antibodies produced in rabbit which are generated by immunization of the rabbit with the peptide having sequence SEQ ID NO. 17 (e.g. coupled to a suitable carrier-protein). The polyclonal antibody (anti-TBP antibody) recognizes the hTBP fusion protein (HA- and His-tag).

An antibody can be applied for Western blot analysis as well as for the affinity purification of TPB fusion protein and of higher order transcription complexes which are associated to the TBP fusion protein. Higher order complexes comprise TAFs which are associated with the TBP fusion protein and TAF-interacting factors.

The description discloses the use of the non-human transgenic animal. A non-human transgenic animal according to the invention can be used for affinity co-purification of higher order transcription complexes, TAFs and TAF-interacting factors from non-human transgenic animal tissue and/or cultured cells of the non-human transgenic animal. Such higher order transcription complexes can be isolated and purified from a variety of different tissues and/or cell types. Preferably, nuclear preparations from such tissue/cell types are performed, most preferably from homogenized cells according to standard protocols (Dignam et al. (1983) Nuc. Acids Res. 11: 1575; Lichtenstein et al. (1987) Cell 51: 963-973; Gorsky et al. (1986) Cell 47: 767-776). If nessecary, nuclear proteins can then be accumulated by standard methods. Therefore, description also discloses methods affinity co-purify higher or to transcription complexes, TAFs and TAF-interacting factors from non-human transgenic animals.

For example, description discloses the affinity-purification of higher-order transcription complexes, TAFs and TAF-interacting factors by using epitope-specific antibodies or charged (positive or negative charged) materials. For example the antibodies already described in detail can be used for this purpose. One of the most preferable co-purification methods for higher order transcription complexes which comprise HA and His epitope-tagged TBPs, preferably hTBPs, include affinity purification using Ni²⁺ and/or anit-HA-antibodies (e.g. commercially available antibodies) and/or antibodies recognizing the epitope according to sequence SEQ ID NO. 17. Such antibodies or Ni²⁺ might be coupled to a suitable column material so that the affinity purification can be performed by using e.g. Ni²⁺-columns or columns with specific antibodies, e.g. with anti-HA antibodies or anti-TBP antibodies.

A TBP fusion protein, the epitope-tagged TBP, is disclosed. Preferably the TBP fusion protein is expressed in a non-human transgenic animal. In particular a hTBP fusion protein is disclosed. A TBP fusion protein can be isolated and purified from a non-human transgenic animal. One embodiment is a HA and His tagged TBP protein, in particular the HA and His tagged hTBP protein. An other embodiment is the protein that has the amino acid sequence SEQ ID NO. 16. In another embodiment the TBP fusion protein comprises one or more cleavage side(s) for a proteinase/peptidase, e.g. a thrombin cleavage site. Preferably the cleavage sites within the amino acid sequence of the fusion protein are located between the epitope-tag(s) and the TBP-protein.

The description discloses a method of preparing a TBP fusion protein, wherein a transgene according to the invention is expressed in a suitable host cell, who is preferably part of a non-human transgenic animal.

Transgenic mRNA and/or a TBP fusion protein encoded by the transgene and/or higher order transcription complexes associated with theTBP fusion protein can be isolated from different types of tissue and/or cell types that are found in the non-human transgenic animal e.g. brain, heart, kidney, liver, lung, nervous system, muscle, glands, bone narrow, cells belonging to the immunsystem, skin etc.

The description discloses the use of the TBP fusion protei, in particular for the isolation of higher order transcription complexes from the non-human transgenic animal and to the characterisation of isolated higher order transcription complexes obtained from different species, from different tissues and/or different cell-types. Therefore, the TBP fusion protein and a non-human transgenic animal according to the invention can be used for the isolation and characterization of individual proteins such as TAFs and TAF-interacting factors which are associated in the different higher order complexes. For example, the proteins assoziated in a particular higher order transcription complex can be dissociated and separated so that individual TAFs and TAF-associated factors can be identified. The composition of TAFs and TAF-interacting factors are different to of least some extend in the different higher order complexes depending on the tissue type and/or the cell type and/or the developmental stage and/or the transgene which is expressed.

TAFs and TAF-interacting factors, in particular tissue-specific factors which have already been characterized and for which antibodies already exist can be quickly identified and assessed for degree of association with the transcription complex. An example of this would be the Bob-I/OCA-B factor, which is thought to be a tissue-specific co-activator responsible for B-cell restricted activation (Gstaiger, M. et al. (1996) EMBO 15, 2781-2790). Although without intrinsic DNA-binding capacity and a requirement for the nearly ubiquitous Oct factors, the tissue-restricted appearance of this factor confers B-cell specific transcriptional activation through a protein-protein mechanism.

Further, novel TAFs and TAF-interacting factors, in particular tissue- and/or cell type-specific and/or developmental (stage) specific and/or cell cycle specific TAFs and TAF-interacting factors, can be identified in a higher order transcription complex. As mentioned above, there is a wealth of data which strongly suggests that many enhancer-binding tissue-specific factors are able to exert influence and, thus, tissue specificity on a gene's transcriptional activity. Therefore "unique", tissue-specific, cell-type specific, cell-cycle specific, developmental stage specific factors might be identified, which regulate the specific expression of genes.

This "universal" transgenic system offers furthermore a powerful tool to investigate the degree to which such TAFs and TAF-interacting factors (e.g. coactivators), associate with TBP and/or TAFs and the transcription complex in a range of tissues and cell types.

The description also discloses a method for the identification and characterization of different higher order transcription complexes, wherein epitope-tagged TBP to which higher order transcription complexes are associated is isolated from a non-human transgenic animal. A method of characterizing the composition of different higher order transcription complexes can for example comprise a) the introduction of a transgene according to the invention into a non-human animal, b) the isolation of the epitope-tagged TBP from different animal tissue and/or different cell types of the animal, optionally at different developmental stages of the animal and c) the determination of the composition of the higher order transcription complexes.

One method of isolating a higher order transcription complex from a transgenic non human animal comprises the affinity purification by using at least one of the epitopes tagged to TBP. Preferably the higher order transcription complex and the TAFs and TAF-interacting factors associated in this complex are co-purified, when the epitope-tagged TBP is isolated. For example, a higher order transcription complex can be isolated, when epitope-tagged TBP is purified by binding of one of ist epitopes, preferably an epitope-tag to a material to which the epitope specifically binds. This material can for example be a Ni²⁺-column (to which a His-epitope binds) or antibodies, e.g. anti-HA antibodies (bind to a HA epitope) or antibodies which bind to an epitope of epitope-tagged TBP that has the sequence SEQ ID NO. 17 or a part thereof (e.g. epitope of sequence SEQ ID NO. 17).

The description also discloses a method of identifying a new and/or a specific TAF and/or TAF-interacting factor. Preferably, a higher order transcription complex is isolated isolated from a non-human transgenic animal according to the invention. Such a method may for example comprise a) the introduction of a transgene according to the invention into a non-human animal, b) the isolation of epitope-tagged TBP from a particular animal tissue and/or a particular cell type of the animal, optionally at a particular developmental stage of the animal and c) dissociation and separation of a TAF and/or a TAF-interacting factor associated with the epitope-tagged TBP in the higher order transcription complex and d) if necessary determination of the aminoacid sequence of the TAF and/or the TAF-interacting factor.

In addition, to corroborating already known mechanisms, the transgenic model has an advantage over current cell culture systems in finding and characterizing new TAFs or TAF-associated factors. As mentioned above, the TAF proteins that have been affinity co-purified to date have come from studies in HeLa and yeast. TAF cDNAs for other organisms have also been found, but with time consuming interaction library screening methods. The "whole organism" aspect of the transgenic model makes this universal transgenic system especially responsive to the recognition of novel tissue-specific activation elements by remaining very "close" to the true in vivo process of transcription.

It is clear that such a tagged-TBP-expressing non-human transgenic animals, e.g. mouse would be able to contribute to the area of drug development. Considerable pharmaceutical interest can be attributed to any 'unique', tissue-specific, cell-type specific, cell-cycle specific, developmental stage specific factors (TAFs, TAF-interacting factors) of a given gene's transcription complex, especially if it is a disease-related gene. The identification and characterization of "key" TAFs and TAF-interacting factors which are involved in transcriptional control of one or a few disease-related genes is a sound strategy to develop therapeutic compounds which alleviate such genetic diseases. Once such a TAF or TAF-interacting factor has been characterized and cloned, any number of screening procedures can be undertaken to identify molecular species/substances which specifically interact with the TAF or TAF-associated factor. A pharmaceutically useful species/substance would be one which enhances or represses the TAF's or TAF-interacting factor's natural activity in vitro and/or in vivo, thus allowing therapeutic manipulation of a related gene.

Identified TAFs and TAF-interacting factors might then also be applied as tools in biochemistry and in molecular biology, for example such proteins from transgenic non-human animals might then be used as probes for the isolation of the corresponding human TAFs and TAF-interacting factors or their cDNAs. Human TAFs and TAF-interacting factors might then also be applied to screen for highly specific new drugs.

### Example 1: Construction of non-human transgenic animals.

### Potential animal sources:

Animals suitable for transgenic experiments were obtained from standard commercial sources, Charles River (Wilmington, MA), Taconic (Germantown, NY), and The Jackson Laboratory (Bar Harbor, Maine). B6SJL/F1 mice were used for embryo retrieval and transfer. B6SJL/F1 males can be used for mating and vasectomized Swiss Webster studs can be used to stimulate pseudopregnancy.

### Transgenic mice:

Female mice six weeks of age are induced to superovulate with a 5 IU injection (0.1 cc, intraperitoneal) of pregnant mare serum gonadotropin (PMSG; e.g. Sigma, Saint Louis, Missouri, USA) followed 48 hours later by a 5 IU injection (0.1 cc, intraperitoneal) of human chorionic gonadotropin (hCG; e.g. Sigma). Females are placed with males immediately after hCG injection. Twenty-one hours after hCG, the mated females are sacrificed by CO₂ asphyxiation or cervical dislocation and embryos are recovered from excised oviducts and placed in M2 media (e.g. Sigma).
Surrounding cumulus cells are removed with hyaluronidase (1 mg/ml). Pronuclear embryos are then washed and placed in M16 media (e.g. Sigma) and then put in a 37° C incubator with a humidified atmosphere at 5% CO₂ O₂, and 90% N₂ until the time of injection.

### Example 2: Preparation of constructs for transfections and microinjections

DNA clones for microinjection were cleaved with appropriate enzymes, DNA clones comprising the MT-hTBP transgene (double-tagged, according to the sequence in table 3) with Cla I and BamHI or EF-hTBP transgene (double-tagged, according to the sequence in table 2) with Eco RI/Eco RI and the appropriate size DNA fragments electrophoresed on 1% agarose gels in TBE buffer (Sambrook et al. (1989)). The DNA bands are visualized by staining with ethidium bromide, excised, and placed in dialysis bags containing 0.3 M sodium acetate, pH 7.0. DNA is electroeluted into the dialysis bags, extracted with phenol-chloroform (1:1), and precipitated by two volumes of ethanol. The DNA is redissolved in TE buffer (10 mM Tris, pH 7.4 and 1 mM EDTA) and purified on a DEAE sephacel (e.g. Pharmacia, Uppsala, Schweden) column. The column is first primed with 0.5 ml of high salt buffer (1.5 M NaCl, 10 mM Tris, pH 7.4, and 1 mM EDTA) followed by washing with 3 ml of low salt buffer (0.15M NaCl, 10mM Tris pH 8.0, and 1 mM EDTA). The DNA solutions are adjusted in salt to 0.15M NaCl then passed through the column to bind DNA to the column matrix. After three washes with 3 ml of low salt buffer, the DNA is eluted in aliquots of 4 x 0.3 ml of high salt buffer and precipitated by two volumes of ethanol. The fractions were pooled by dissolving in 200µl of TE, phenol:chloroform extracted once, chloroform extracted twice, then the DNA was precipitated with ethanol overnight. The DNA was resuspended in microinjection buffer TE (10mM Tris pH 7.4, 0.1 mM EDTA) and the DNA concentration was adjusted to 2ng/µl and visualized against known DNA standards by electorphoresing on an agarose gel.

### Microinjection:

DEAE purified transgene DNA (MT-hTBP or EF-hTBP) was dissolved in microinjection buffer at 2ng/µl. Microneedles and holding pipettes were pulled on a Flaming Brown micropipette puller e.g. Model P87 (Sutter Inst. Co.). Holding pipettes were then broken and fire poished to on a deFonbrune-type microforge (e.g. Technical Product Inst. Inc.). Pipettes were mounted micromanipulators (e.g. on Leitz) which were attached to a Zeiss Axiovert® 135 microscope. The air-filled injection pipette (e.g. Medical System Corp.) was filled with DNA solution through the tip. Embryos in groups of 40-50 were placed in 200µl of M2 media under silicone oil for micromanipulation. The embryo was oriented and held with the holding pipette and then the injection pipette was inserted into the pronucleus closest to the injection pipette. The injection was monitored by the swelling of the pronucleus. Following injection, the group of embryos was placed in M16 media until transfer to recipient females.

### Example 3: Embryo transfer by microinjection.

Randomly cycling adult female mice are paired with vasectomized males. Swiss Webster or other comparable strains can be used for this purpose. Recipient females are mated at the same time as donor females. At the time of embryo transfer, the recipient females are anesthetized with an intraperitoneal injection of 0.015 ml of 2.5% avertin per gram of body weight. The oviducts are exposed by a single midline dorsal incision. An incision is then made through the body wall directly over the oviduct. The ovarian bursa is then torn with watchmakers forceps. Embryos to be transferred are placed in M16 and then in the tip of a transfer pipette (about 10-12 embryos). The pipette tip is inserted into the infundibulum and the embryos transferred. After the transfer, the incision is closed by two sutures and the skin stapled. The recipient recovered for three hours on a warming tray then was placed in the colony for delivery.

A total of 469 pronuclear embryos were micronjected for MT-hTBP and 170 pups were born and for EF-hTBP a total 407 pronuclear embryos yield 76 pups.

### Example 4: Detection of transgenic DNA in founder mice.

### 4a) DNA Extraction:

Genomic DNA of possible founder mice was extracted from a small piece of tail tissue (ca. 1 cm) cut from 2-4 week old offspring. Tail sections were incubated overnight on a shaker in 500-750 µl Tail Buffer at 54°C (Tail Buffer: 10mM Tris pH 7.5, 100mM NaCl, 10mM EDTA, 0.5% SDS, 30µg/ml Proteinase K). To each tail sample was subsequently added an equivolume of phenol/chloroform/isoamyl alcohol (25:24:1). Samples were shaken gently by hand or automatically on a Vortex micer with low setting. Samples were all centrifuged in tabletop centrifuge for 10 minutes. Aqueous phase was transferred to 5ml polypropylene tube (e.g. falcon tube®). An equivolume of ethanol was slowly added (dropwise) to the tube to allow the DNA to gradually precipitate. A second volume of ethanol was added forcefully to mix the contents of the tube. Tubes were then inverted several times to ensure mixture. Genomic DNA was then spooled around a flat pipette-tip (sequencing-gel tip) and transferred to an individual well of a microtiter dish. DNA was then air dried in dish for 4 hours. 200µl of 1x TE was then added to each well (1x TE: 10mM Tris pH 7.4, 1 mM EDTA). Genomic DNA was then allowed to dissolve for 15-30 minutes at room temperature, then mixed gently by carefully pipetting up and down. Microtiter plates were often stored at -20°C before PCR testing. Prior to testing, 10µl were removed and digested with EcoRl (e.g. 10µl genomic DNA, 2µl 10x EcoRI buffer, 7µl H₂O, 1µl EcoRI; enzyme and buffer from Boehringer-Mannheim, Mannheim, Germany).

### 4b) PCR Reactions:

Digested genomic DNA was diluted 1:3 with water. Samples were then heated to 100°C on a heating block for 10 minutes. 2µl was then used for PCR reactions. Reactions were carried out in a 50µl volume consisting of 2µl genomic DNA, 1x reaction buffer, 1.5mM MgCl, 0.8µM forward oligonucleotide primer, 0.8µM reverse oligonucleotide primer, 8µl of nucleotide mixture containing 0.2mM of each nucleotide (dATP, dCTP, dTTP, and dGTP), and 2.5 units of Taq polymerase. PCR was performed using e.g. AmpliTaq® enzyme and buffer from Perkin Elmer (Perkin Elmer Norwalk, Conneticut, USA).

Detection of the MT-hTBP transgene was accomplished using the forward primer oligonucleotide (sense primer) 5' GGAGCA ACC GCC TGC TGG GTG C 3' (SEQ ID NO. 5) and the reverse primer oligonucleotide (antisense primer) 5' CCT GTG TTG CCT GCT GGG ACG 3' (SEQ 10 NO.6).

Detection of the EF-hTBP transgene was accomplished, with the forward - oligonucleotide primer 5' GGA GAC TGA AGT TAG GCC AGC 3'(SEQ ID NO.7). The same reverse primer as in the MT-hTBP detection was used (5' CCT GTG TTG CCT GCT GGG ACG 3').

Temperature cycling was carried forth using a robotic temperature cycler (e.g. from Stratagene, La Jolla, CA, USA).

The cycling temperatures were:

| | | | |
|---|---|---|---|
| cycle 1: | 94°C 5min | 60°C 3min | 72°C 2min; |
| cycle 2-25: | 94°C 1 min | 60°C 2min | 72°C 3min; |
| cycle 26: | 94°C 1 min | 60°C 2min | 72°C 5min. |

or

| | | | |
|---|---|---|---|
| cycle 1-40: | 95°C 2 min | 55°C 1 min | 72°C 1 min. |

The presence of amplified product was detected by running standard agarose gel electrophoresis (e.g. 1.2-1,5A agarose) and staining the gel with ethidium bromide for visualization of DNA with UV light.

Positive MT-hTBP samples were distinguished by the presence of a about 580 bp amplified DNA product. Positive EF-hTBP samples produced a about 500bp amplified DNA fragment.

The DNA-analysis from 170 pups for MT-hTBP by PCR-reaction indicated that 35 genomic DNA-samples were positive for the transgene. The analysis of the genomic DNA of the 76 EF-hTBP pubs by PCR analysis indicated that the EF-hTBP transgene was contained in 9 mice.

### 4d) Transgenic model expansion:

DNA positive Go founders were bred to non-transgenic B6SJL mates and the resulting litters were PCR genotyped. G₁ offspring were used for continued breeding and maintainance of the individual lines and for further mRNA and protein expression analysis.

### Example 5: Detection of transgenic mRNA by S1 nuclease protection assay:

### 5a) Specifics:

Oligonucleotides (oligo) complementary to the 5' end and the 3' end of the transgenic transcript were produced:

Underlined regions are non-hybridizincing sequence. 40ng of oligo were 5'-labeled with (³²P gamma)ATP (5000cpm/mM) using T4 polynucleotide kinase and buffer e.g. from Boehringer-Mannheim-(Mannheim). The reaction was performed in 50µl reaction volume at 30°C for 1 hour. Labeled oligo was isolated from unincorporated (³²P gamma)ATP using size exclusion chromatography (e.g. Push-Columns®, Stratagene, La Jolla, CA, USA).

### 5b) Promoter induction:

Prior to RNA analysis, it was necessary to induce the MT-hTBP mice, since the promoter is activated in the presence of Zn²⁺. On each MT-hTBP mouse, two interperitoneal injections of ZnSO₄ in H₂O were made at 18 hours and again at 4 hours prior to liver removal (dose was 0.1mg ZnSO₄/10g mouse wight).

### 5c) RNA Extraction:

Total RNA was extracted from 1-5g of tissue using commercially available Trizol reagent (e.g. Gibco-BRL, Paisly, UK). Tissue was homogenized with an Ultra-Turrax in 5ml of Trizol solution in a 12ml polypropylene tube (e.g. falcon tube®). 1ml chloroform was added, and tubes were capped and shaken vigorously by hand for 15 seconds. Solutions were incubated at RT for 3 min, then centrifuged at 12,000xg for 15 mins at 4°C in Sorvall SS-34 rotor. Supernatant was transferred to a new tube; 2.5ml isopropanol was added and mixed. Incubation at RT for 10 min followed. The samples were centrifuged with 12,000xg for 10 min at 4°C. Supernatant was removed and RNA pellet were washed with 5ml 75% ethanol. Samples were mixed and centrifuged at 7,500xg for 10 min. RNA pellets were resuspended in RNAse-free water and subsequently quantitated by measuring the absorbance at 260nm.

### 5d) S1 Nuclease protection:

Uniform amounts of RNA (10-20µg) were brought up to 100µl with RNAse-free water. 50,000-150,000 dpm of labeled oligo was added (0.1-1ng, depending on labeling efficiency). RNA/oligo mixture was precipitated with 0.3M sodium acetate and ethanol. The RNA/oligo pellet was washed and air dried. 23µl hybridization solution was added (80% formamide, 10mM PIPES pH 6.4 (Sambrook et al. (1989)), 1mM EDTA, 0.05% SDS). Reaction was mixed and denatured at 65°C for 20 min. and then 2µl of 5M NaCl werw added to each sample at 65°C. Samples were incubated 1 additional hour at 65°C in a H₂O bath, then the temperature of the bath was reset to 37°C. Gradual temperature decrease from 65°C to 37°C (overnight) facilitated the specific oligo-mRNA hybridization. The next day, 300µl S1 buffer were added to each sample (S1 buffer. 167U/ml S1 nuclease (e.g. Gibco BRL, Paisly, UK), 0.3M NaCl, 30mM NaOAc (pH 4.5), 3mM ZnSO₄. Samples were incubated at room temperature for 1 hour, then 1 ml ethanol was added (cold) to precipitate all nucleic acid. The pellet was centrifuged and washed and dried briefly in a Speed-Vac. Pellet was then resuspended in 12µl S1 loading buffer (85% formamide, 0.01% bromphenol blue, 0.01% xylene cyanol, 1x TBE). Samples were heated to 70°C for 5 min before being loaded and size separated using denaturing (6M urea) thin polyacrylamide gel electrophoresis. Autoradiography of the dried gel facilitated detection of protected, undigested bands.

From 35 founders for MT-hTBP, 7 founders showed detectable mRNA levels as analyzed by S1 protection assay. For EF-hTBP, 3 founders showed detectable mRNA from S1 analysis.

### Example 6: Northern blot detection of transgenic mRNA.

### 6a) Synthesis of the hybridization probe by PCR amplification of TBP and labeling of the TBP probe:

Oligonucleotides/primers bracketing a 498bp fragment of the double-tagged hTBP construct were designed and synthesized.

The forward oligo began 10 bases downstream from "AUG" start site (ATG site in Table 1). The sequence of the sense primer was:
SEQ ID NO. 10: 5' CCCTATGACGTCCCGGATTACG 3'.

The reverse primer ended at 507 bp downstream of the "AUG" start site (ATG site in Table 1). The sequence of the antisense primer was:
SEQ ID NO. 11: 5' GTGGAGTGGTGCCCGGCAAGGG 3'.

PCR reactions were carried out with 0.2µg pAG-17, 0.5mM MgCl₂. 0-8µM of each primer, 1x PCR buffer (Perkin-Elmer), 0.2mM dATP, dTTP, dCTP, dGTP. and 2.5U of AmpliTaq® enzyme (Perkin Elmer Norwalk, Conneticut, USA).

Thermocycler program:

| | | | |
|---|---|---|---|
| cycles 1-35: | 94°C 1 min | 55°C 1min | 72°C 1min; |

then 10 min at 72°C, then 4°C storage. The amplified bands were purified from a 0.7% agarose gel.

The TBP-DNA- probe was labeled with random primers and Klenow fragment enzyme from Megaprime labeling kit® (Amersham, UK). 25-50ng probe DNA was combined with 5µl random hexamer primer (e.g. Amersham) and 20 µl H₂O. DNA was denatured at 100°C for 5 minutes. Labeling mix was added to a final volume of 50µl with 1 x reaction buffer, 1 x dATP, 1x dTTP, 1 x dGTP, 4µl 3000Ci/mmol alpha-³²P dCTP (e.g. Amersham) and 2U Klenow enzyme. Reaction was allowed to proceed at RT for 1 hour. Labeled DNA was isolated from unincorporated nucleotides using size exclusion chromatography (e.g. Push Columns®).

### 6b) Gel electrophoresis and transfer:

10-20µg of total RNA was ethanol precipitated, the pellet was denatured 10 min at 65°C in RNA loading buffer (65% formamide, 20% formaldehyde (37% solution), 1x MOPS buffer (Sambrook et al. (1989)), 5% glycerol, 0.01% bromphenol blue, 0.01% xylene cyanol, 0.1 mg/ml ethidium bromide). RNA was size fractionated on 1% agarose gel containing 18% formaldehyde (37% solution) and 1x MOPS running buffer (40mM MOPS pH 7.0, 10mM sodium acetate, 1 mM EDTA). Gels were run slowly overnight at 1V/cm in 1xMOPS running buffer containing 18% formaldehyde (37% solution). Gel was then treated in 0.05M NaOH/1.5M NaCl for 30 minutes, followed by 20 minutes in 0.5M Tris (pH 7.4)/1.5M NaCl. RNA was transferred onto nylon membrane (e.g. Hybond-N+, Amersham, UK) using capillary techniques. Membrane-bound RNA was crosslinked, e.g. UV-crosslinked using a Stratalinker® (Stratagene, La Jolla, CA, USA).

### 6c) Hybridization and washing:

Pre-hybridization was perfomred for example in "Rapid-Hyb" buffer (Amersham, UK) in rotating hybridization oven (e.g. Hybaid) at 65°C for 1-2 hours. Hybridization was performed for example in 6-10 ml "Rapid-Hyb" buffer containing 10⁶ -10x10⁶ cpm of denatured probe at 65°C for 2-3 hours. Membrane was washed twice at RT (10 min/wash) in 2x SSC/0.1%SDS (1xSSC: 150mM NaCl, 15mM Na₃citrate, pH 7.0). Membrane was further washed 2 times at 65°C (15min/wash) in 1xSSC/0.1% SDS. The next 2 washs were performed at 65°C for 15 min in 0.5xSSC/0.1%SDS, followed by 1 or 2 final washes (as necessary) for 15 min/wash at 65°C in 0.2xSSC/0.1% SDS. Autoradiography of washed membrane.

### Example 7: Generation of Polyclonal Antibody Specific for tagged-hTBP

### General Strategy:

A significant amount of sequence homology exists between the mouse and human TBP. Therefore, most commercially available antibodies will cross react, detecting both endogenous and transgenic TBP. To differentiate between the two, a polyclonal antibody was generated against the tagged region of the transgenic TBP (corresponding to the thrombin cleavage site and the His tag of the tagged hTBP):
SEQ ID NO. 12: H₂N- MGSSHHHHHHSSGLVPRGC-COOH

This peptide was coupled to carrier protein and injected into rabbits using standard protocols. Serum was collected at regular intervals and tested for anti-hTBP titer using ELISA assays.

### Example 8: Western-Blot Detection of Transgenic Protein:

### 8a) Preparation of liver nuclear extracts:

MT-hTBP mice were subjected to 2 intraperitoneal injections of ZnSO₄ (see 5b)). Mice were killed with cervical dislocation and livers removed. Livers were homogenized immediately in 10ml homogenization buffer (1.8M sucrose, 10mM HEPES pH 7.4 (Sambrook et al. (1989), 25mM KCL, 1mM EDTA, 5% glycerol, 0.15 mM spermine, 0.5mM spermidine, 0.4mM PMSF). After homogenization, volume was increased to 25 ml with the same buffer. The homogenate was carefully layered onto 7 ml homogenization buffer in centrifuge tubes, e.g. in Ultra-Clear SW-28 tubes® (Beckman, Palo Alto, CA, USA). Samples were centrifuged in SW-28 rotor for 1 hour at 25,000 rpm (4°C). Supernatant was carefully removed, and pelleted nuclei were resuspended in 200µl NEXB buffer (20mM HEPES pH 7.9, 400mM NaCl, 1 mM EDTA. 1mM EGTA, 1mM DTT, 1 mM PMSF, 10% glycerol, 2µg/ml aprotinin, 2µg/ml leupeptin, 2µg/ml pepstatin-A). Resuspendet nuclei were incubated on ice for 15-30 min with frequent mixing and pipetting up and down. Samples were submitted to 5 freeze/thaw cycles with dry ice/ethanol bath and 37°C H₂O bath. Samples were centrifuged for 30 seconds at high speed and supernatant was measured for protein content, e.g. with protein assay reagent (e.g. Bio-Rad, Hercules, -CA, USA).

### 8b) Electrophoresis and transfer:

20-75µg of extract was size separated by denaturing gel electrophoresis. Resolving gel: 10% acrylamide (1:37 ratio acrylamide:bis-acrylamide), 0.1% SDS, 375mM Tris pH 8.8. Stacking gel: 5% acrylamide, 0.1% SDS, 125mM Tris pH 8.3. Running buffer: 25mM Tris pH 8.3, 192mM glycine, 0.1% SDS. Protein was transferred onto pure nitrocellulose membrane (e.g. Bio-Rad) with semi-dry blotter (e.g. Hoefer, San Francisco, CA, USA) using modified Bjerrum transfer buffer (48mM Tris, 39mM glycine, 10% methanol, 0.0375% SDS, pH 9.2). Transfer was allowed to run at 0.8mA/cm for 2-4 hours.

### 8c) Immunodetection:

Membranes were blocked 1-2 hours at room temperature on rocking surface in 1x TBS (20mM Tris pH 7.5, 500mM NaCl) with 3% gelatin (e.g. Bio-Rad), Membranes were washed 10 min in 1xTTBS (1xTBS with 0.05% Tween-20) at RT. Hybridization with primary antibody in 1% gelatin/1xTTBS was performed for 4 hours to overnight on rocking surface at RT. Membranes were washed 2-3 times (5 min/wash) with 1x TTBS. Hybridization with secondary antibody coupled with alkaline phosphatase (AP) in 1 % gelatin/1xTTBS was done for 2-3 hours. Membranes were washed again 2-3 times (5 min/wash) in 1xTTBS at RT followed by 5 min wash in 1x TBS buffer.
Membranes were then incubated in 1x development buffer (e.g. BioRad) containing NBT/BCIP reagents at RT until sufficient appearance of bands occured. AP reaction was stopped by H₂O wash.
In the examples and the application text
Table 1 corresponds to SEQ ID NO.13, table 2 corresponds to SEQ ID NO 14, table 3 corresponds to SEQ ID NO. 15, table 4 corresponds to SEQ ID NO. 16 and table 5 corresponds to SEQ ID NO. 17.
TBP may have a sequence that corresponds to the Sequence SEQ ID NO 14 (nucleotides 2530 to 3550) or a sequence that corresponds to SEQ ID NO 15, nucleotides 1193 to 2213. This sequence can be connected to an epitope that corresponds to nucleotides 2434-2529 in SEQ ID NO.14 or to nucleotides 1097-1192 of SEQ ID NO 15.

## Claims

1. Transgenic non-human animal having the ability to overexpress epitope-tagged TATA-box binding protein (TBP).

2. Transgenic non-human animal according to claim 1, wherein TBP is expressed as fusion protein with two epitope-tags.

3. Transgenic non-human animal according to one or more of claims 1 and 2, wherein the fusion protein comprises human TBP (hTBP).

4. Transgenic non-human animal according to one or more of claims 1 to 3, wherein the fusion protein comprises a HA- and a His-epitope.

5. Transgenic non-human animal according to claim 4, wherein the fusion protein has the sequence SEQ ID NO. 16.

6. Transgenic non-human animal according to one or more of claims 1 to 5, wherein the transgenic animal is a mouse.

7. A method of making a transgenic non-human animal as claimed in one or more of claims 1 to 6 by introducing a transgene into the germline and/or into somatic cells of a non-human animal.

8. A method of making a non-human transgenic animal as claimed in claim 7 by microinjecting transgenic DNA into a non-human animal zygote.

9. A method of making a non-human transgenic animal as claimed in claim 7 by transfecting a non-human blastocyst with a vector that contains a transgene.

10. A method of making a non-human transgenic animal as claimed in claim 7 by introducing a transgene into an non-human embryonic stem cell.

## Patentansprüche

1. Transgenes, nichtmenschliches Tier mit der Fähigkeit, ein epitopmarkiertes TATA-Box-Bindeprotein (TBP) zu überexprimieren.

2. Transgenes, nichtmenschliches Tier nach Anspruch 1, wobei TBP als Fusionsprotein mit zwei Epitop-Tags exprimiert wird.

3. Transgenes, nichtmenschliches Tier nach einem oder mehreren der Ansprüche 1 und 2, wobei das Fusionsprotein humanes TBP (hTBP) umfasst.

4. Transgenes, nichtmenschliches Tier nach einem oder mehreren der Ansprüche 1 bis 3, wobei das Fusionsprotein ein HA- und ein His-Epitop umfasst.

5. Transgenes, nichtmenschliches Tier nach Anspruch 4, wobei das Fusionsprotein die Sequenz von SEQ ID NR: 16 aufweist.

6. Transgenes, nichtmenschliches Tier nach einem oder mehreren der Ansprüche 1 bis 5, wobei das transgene Tier eine Maus ist.

7. Verfahren zur Herstellung eines transgenen, nichtmenschlichen Tieres, wie in einem oder mehreren der Ansprüche 1 bis 6 beansprucht, durch Einführen eines Transgens in die Keimbahn und/oder in somatische Zellen eines nichtmenschlichen Tieres.

8. Verfahren zur Herstellung eines nichtmenschlichen, transgenen Tieres, wie in Anspruch 7 beansprucht, durch Mikroinjizieren von transgener DNA in eine Zygote eines nichtmenschlichen Tieres.

9. Verfahren zur Herstellung eines nichtmenschlichen, transgenen Tieres, wie in Anspruch 7 beansprucht, durch Transfizieren einer nichtmenschlichen Blastozyste mit einem Vektor, der ein Transgen enthält.

10. Verfahren zur Herstellung eines nichtmenschlichen, transgenen Tieres, wie in Anspruch 7 beansprucht, durch Einführen eines Transgens in eine nichtmenschliche embryonale Stammzelle.

## Revendications

1. Animal transgénique non humain ayant la capacité de surexprimer une protéine de liaison (TBP) marquée par un épitope TATA-box.

2. Animal transgénique non humain selon la revendication 1, dans lequel TBP est exprimé comme protéine de fusion à deux epitope-tags.

3. Animal transgénique non humain selon une ou plusieurs des revendications 1 et 2, dans lequel la protéine de fusion comprend du TBP (hTBP) humain.

4. Animal transgénique non humain selon une ou plusieurs des revendications 1 à 3, dans lequel la protéine de fusion comprend un épitope HA- et un épitope His-.

5. Animal transgénique non humain selon la revendication 4, dans lequel la protéine de fusion présente la séquence SEQ ID NO. 16.

6. Animal transgénique non humain selon une ou plusieurs des revendications 1 to 5, dans lequel l'animal transgénique est une souris.

7. Un procédé d'obtention d'un animal transgénique non humain tel que revendiqué dans une ou plusieurs des revendications 1 à 6, par introduction d'un transgène dans la lignée germinale et/ou dans les cellules végétatives d'un animal non humain

8. Un procédé d'obtention d'un animal transgénique non humain tel que revendiqué dans la revendication 7, par micro-injection d'ADN transgénique dans un zygote d'un animal non humain.

9. Un procédé d'obtention d'un animal transgénique non humain tel que revendiqué dans la revendication 7, par transfection d'un blastocyste non humain par un vecteur qui contient un transgène.

10. Un procédé d'obtention d'un animal transgénique non humain tel que revendiqué dans la revendication 7, comprenant l'introduction d'un transgène dans la cellule souche embryogénique non humaine.
